# EUROPEAN PATENT APPLICATION

(11) **EP 4 446 343 A1**
(43) Date of publication of application: **16.10.2024**
(21) Application number: 22904212.2
(22) Date of filing: 06.12.2022
(51) Int. Cl.: C07K 16/28, A61K 39/395, A61P 9/14, A61P 29/00, A61P 35/00, C12N 1/15, C12N 1/19, C12N 1/21, C12N 5/10, C12N 15/12, C12N 15/13

(54) **ANTIBODY OR ANTIGEN-BINDING FRAGMENT THEREOF**

(30) Priority: 07.12.2021 JP 2021198818
(71) Applicant: OSAKA UNIVERSITY, Suita-shi Osaka 565-0871 (JP); Periotherapia Co., Ltd., Suita-shi, Osaka 565-0871 (JP)
(72) Inventor: TANIYAMA, Yoshiaki, Suita-shi, Osaka 565-0871 (JP); SANADA, Fumihiro, Suita-shi, Osaka 565-0871 (JP); MORISHITA, Ryuichi, Suita-shi, Osaka 565-0871 (JP)
(74) Representative: Hoffmann Eitle
(86) International application number: PCT/JP2022/044857
(87) International publication number: WO 2023/106284

(57) **Abstract**

An object is to provide a novel antibody or antigen-binding fragment thereof against exon 21 of human periostin. The object is achieved by an antibody or antigen-binding fragment thereof bindable to a conformational epitope on human periostin, wherein the epitope contains the amino acid sequence A represented by SEQ ID NO: 61 and the amino acid sequence B represented by any one of SEQ ID NOs: 62 to 64.

## Description

### Technical Field

The present invention relates to an antibody or antigen-binding fragment thereof.

### Background Art

Periostin is an extracellular matrix protein composed of a polypeptide of a molecular weight of about 90,000. The polypeptide chain contains a signal sequence, a cysteine-rich domain, a four-fold repeated domain, and a C-terminal domain. It has been suggested that periostin gene expression is associated with vascular restenosis conditions, cancer, inflammation, and angiogenesis conditions.

Patent Literature 1 reports an antibody against exon 21 of periostin, and states that the antibody can inhibit periostin, which has cell adhesion activity; inhibit the action of a specific periostin variant that is increasingly expressed in intimal hypertrophy, cancer, inflammations including inflammatory colitis, diseases involving angiogenesis etc.; and limit the exacerbation of disease conditions to treat the diseases.

### Citation List

### Patent Literature

Patent Literature 1: WO2014/136910A

### Summary of Invention

### Technical Problem

In the course of research, the present inventors found that existing antibodies against exon 21 of periostin have a very limited binding ability to human periostin secreted from human cells. Thus, they focused on the need to develop novel antibodies that would improve this problem.

An object of the present invention is to provide a novel antibody against exon 21 of human periostin or an antigen-binding fragment thereof.

### Solution to Problem

The present inventors conducted extensive research and found that the object can be achieved by an antibody or antigen-binding fragment thereof that is bindable to a conformational epitope on human periostin that contains the amino acid sequence A represented by SEQ ID NO: 61 and the amino acid sequence B represented by any one of SEQ ID NOs: 62 to 64. The inventors conducted further research based on this finding and completed the present invention. Specifically, the present invention includes the following subject matter.
Item 1. An antibody or antigen-binding fragment thereof bindable to a conformational epitope on human periostin,
   the epitope comprising the amino acid sequence A represented by SEQ ID NO: 61 and the amino acid sequence B represented by any one of SEQ ID NOs: 62 to 64.
Item 2. The antibody or antigen-binding fragment thereof according to Item 1, wherein the human periostin is human periostin secreted from a human cell.
Item 3. The antibody or antigen-binding fragment thereof according to Item 1 or 2, wherein the human cell is a human breast cancer cell.
Item 4. The antibody or antigen-binding fragment thereof according to any one of Items 1 to 3, which is a monoclonal antibody or antigen-binding fragment thereof.
Item 5. The antibody or antigen-binding fragment thereof according to any one of Items 1 to 4, comprising
   heavy-chain CDR 1 comprising the amino acid sequence represented by SEQ ID NO: 1 and/or 2, SEQ ID NO: 13 and/or 14, SEQ ID NO: 25 and/or 26, SEQ ID NO: 37 and/or 38, or SEQ ID NO: 49 and/or 50,
   heavy-chain CDR 2 comprising the amino acid sequence represented by SEQ ID NO: 3 and/or 4, SEQ ID NO: 15 and/or 16, SEQ ID NO: 27 and/or 28, SEQ ID NO: 39 and/or 40, or SEQ ID NO: 51 and/or 52,
   heavy-chain CDR 3 comprising the amino acid sequence represented by SEQ ID NO: 5 and/or 6, SEQ ID NO: 17 and/or 18, SEQ ID NO: 29 and/or 30, SEQ ID NO: 41 and/or 42, or SEQ ID NO: 53 and/or 54,
   light-chain CDR 1 comprising the amino acid sequence represented by SEQ ID NO: 7 and/or 8, SEQ ID NO: 19 and/or 20, SEQ ID NO: 31 and/or 32, SEQ ID NO: 43 and/or 44, or SEQ ID NO: 55 and/or 56,
   light-chain CDR 2 comprising the amino acid sequence represented by SEQ ID NO: 9 and/or 10, SEQ ID NO: 21 and/or 22, SEQ ID NO: 33 and/or 34, SEQ ID NO: 45 and/or 46, or SEQ ID NO: 57 and/or 58, and
   light-chain CDR 3 comprising the amino acid sequence represented by SEQ ID NO: 11 and/or 12, SEQ ID NO: 23 and/or 24, SEQ ID NO: 35 and/or 36, SEQ ID NO: 47 and/or 48, or SEQ ID NO: 59 and/or 60.
Item 6. The antibody or antigen-binding fragment thereof according to any one of Items 1 to 5, comprising the following (A), (B), (C), (D), or (E) :
   (A) heavy-chain CDR 1 comprising the amino acid sequence represented by SEQ ID NO: 1 and/or 2,
      heavy-chain CDR 2 comprising the amino acid sequence represented by SEQ ID NO: 3 and/or 4,
      heavy-chain CDR 3 comprising the amino acid sequence represented by SEQ ID NO: 5 and/or 6,
      light-chain CDR 1 comprising the amino acid sequence represented by SEQ ID NO: 7 and/or 8,
      light-chain CDR 2 comprising the amino acid sequence represented by SEQ ID NO: 9 and/or 10, and
      light-chain CDR 3 comprising the amino acid sequence represented by SEQ ID NO: 11 and/or 12;
   (B) heavy-chain CDR 1 comprising the amino acid sequence represented by SEQ ID NO: 13 and/or 14,
      heavy-chain CDR 2 comprising the amino acid sequence represented by SEQ ID NO: 15 and/or 16,
      heavy-chain CDR 3 comprising the amino acid sequence represented by SEQ ID NO: 17 and/or 18,
      light-chain CDR 1 comprising the amino acid sequence represented by SEQ ID NO: 19 and/or 20,
      light-chain CDR 2 comprising the amino acid sequence represented by SEQ ID NO: 21 and/or 22, and
      light-chain CDR 3 comprising the amino acid sequence represented by SEQ ID NO: 23 and/or 24;
   (C) heavy-chain CDR 1 comprising the amino acid sequence represented by SEQ ID NO: 25 and/or 26,
      heavy-chain CDR 2 comprising the amino acid sequence represented by SEQ ID NO: 27 and/or 28,
      heavy-chain CDR 3 comprising the amino acid sequence represented by SEQ ID NO: 29 and/or 30,
      light-chain CDR 1 comprising the amino acid sequence represented by SEQ ID NO: 31 and/or 32,
      light-chain CDR 2 comprising the amino acid sequence represented by SEQ ID NO: 33 and/or 34, and
      light-chain CDR 3 comprising the amino acid sequence represented by SEQ ID NO: 35 and/or 36;
   (D) heavy-chain CDR 1 comprising the amino acid sequence represented by SEQ ID NO: 37 and/or 38,
      heavy-chain CDR 2 comprising the amino acid sequence represented by SEQ ID NO: 39 and/or 40,
      heavy-chain CDR 3 comprising the amino acid sequence represented by SEQ ID NO: 41 and/or 42,
      light-chain CDR 1 comprising the amino acid sequence represented by SEQ ID NO: 43 and/or 44,
      light-chain CDR 2 comprising the amino acid sequence represented by SEQ ID NO: 45 and/or 46, and
      light-chain CDR 3 comprising the amino acid sequence represented by SEQ ID NO: 47 and/or 48; or
   (E) heavy-chain CDR 1 comprising the amino acid sequence represented by SEQ ID NO: 49 and/or 50,
      heavy-chain CDR 2 comprising the amino acid sequence represented by SEQ ID NO: 51 and/or 52,
      heavy-chain CDR 3 comprising the amino acid sequence represented by SEQ ID NO: 53 and/or 54,
      light-chain CDR 1 comprising the amino acid sequence represented by SEQ ID NO: 55 and/or 56,
      light-chain CDR 2 comprising the amino acid sequence represented by SEQ ID NO: 57 and/or 58, and
      light-chain CDR 3 comprising the amino acid sequence represented by SEQ ID NO: 59 and/or 60.
Item 7. The antibody or antigen-binding fragment thereof according to any one of Items 1 to 6, comprising
   (A)
      heavy-chain CDR 1 comprising the amino acid sequence represented by SEQ ID NO: 1 and/or 2,
      heavy-chain CDR 2 comprising the amino acid sequence represented by SEQ ID NO: 3 and/or 4,
      heavy-chain CDR 3 comprising the amino acid sequence represented by SEQ ID NO: 5 and/or 6,
      light-chain CDR 1 comprising the amino acid sequence represented by SEQ ID NO: 7 and/or 8,
      light-chain CDR 2 comprising the amino acid sequence represented by SEQ ID NO: 9 and/or 10, and
      light-chain CDR 3 comprising the amino acid sequence represented by SEQ ID NO: 11 and/or 12.
Item 8. An antibody obtained by a method comprising selecting an antibody bindable to human periostin secreted from a human breast cancer cell from among antibodies produced by a hybridoma obtained from an animal immunized with at least one protein or peptide selected from the group consisting of:
   (e) a protein or peptide containing the amino acid sequence represented by SEQ ID NO: 65, and
   (f) a protein or peptide containing an amino acid sequence X with at least 85% identity to the amino acid sequence represented by SEQ ID NO: 65 and containing the amino acid sequence A represented by SEQ ID NO: 61 and the amino acid sequence B represented by any one of SEQ ID NOs: 62 to 64 within the amino acid sequence X.
Item 9. An antibody produced from a hybridoma obtained from an animal immunized with at least one protein or peptide selected from the group consisting of:
   (e) a protein or peptide containing the amino acid sequence represented by SEQ ID NO: 65, and
   (f) a protein or peptide containing an amino acid sequence X with at least 85% identity to the amino acid sequence represented by SEQ ID NO: 65 and containing the amino acid sequence A represented by SEQ ID NO: 61 and the amino acid sequence B represented by any one of SEQ ID NOs: 62 to 64 within the amino acid sequence X,
   the antibody being bindable to human periostin secreted from a human breast cancer cell.
Item 10. A polynucleotide comprising a coding sequence of the antibody or antigen-binding fragment thereof of any one of Items 1 to 9.
Item 11. A cell comprising the polynucleotide of Item 10.
Item 12. A conjugate of the antibody or antigen-binding fragment thereof of any one of Items 1 to 9 with a drug.
Item 13. A pharmaceutical composition comprising at least one member selected from the group consisting of the antibody or antigen-binding fragment thereof of any one of Items 1 to 9 and the conjugate of Item 12.
Item 14. The pharmaceutical composition according to Item 13, for use in the prevention or treatment of cancer, for use in the inhibition of vasculogenic mimicry in cancer, for use in the inhibition of periostin, for use in the prevention or treatment of inflammation-related diseases, for use in the inhibition of intimal hypertrophy, for use in the inhibition of angiogenesis, or for use in the prevention or treatment of aneurysm.
Item 15. A reagent comprising at least one member selected from the group consisting of the antibody or antigen-binding fragment thereof of any one of Items 1 to 9 and the conjugate of Item 12.

### Advantageous Effects of Invention

The present invention provides a novel antibody or antigen-binding fragment thereof against exon 21 of human periostin.

### Brief Description of Drawings

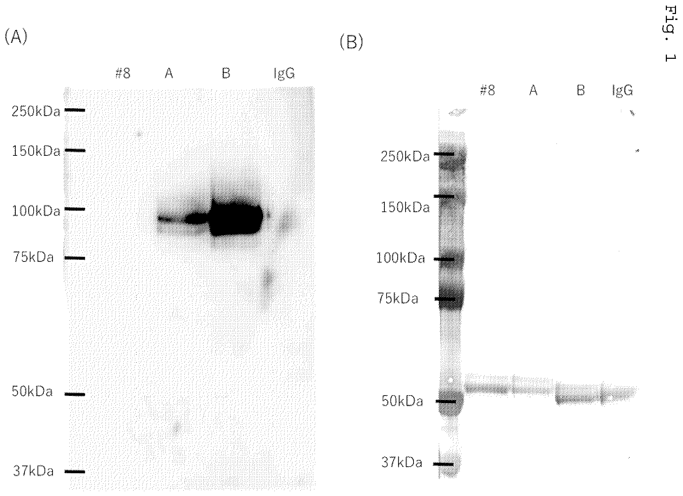

Fig. 1 shows the results of a human periostin-exon 21 recognition test with each antibody (Test Example 2). Fig. 1(A) shows the results of western blotting of a sample after immunoprecipitation. Fig. 1(B) shows a CBB-stained gel after western blotting. The antibody used is indicated at the top of each photograph, and the molecular weight of each band of molecular weight markers is indicated on the left.

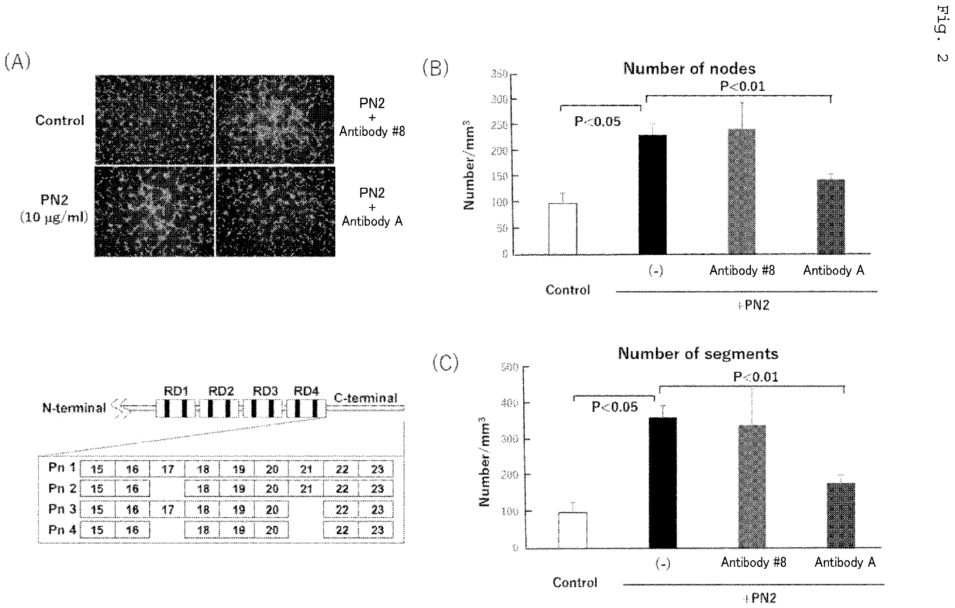

Fig. 2 shows the results of Test Example 5, which examined the effect of each antibody on vasculogenic mimicry. Fig. 2(A) shows fluorescent photographic images of vasculogenic mimicry models of a cultured cell line. The fluorescent areas indicate 4T07 mouse breast cancer cells. Fig. 2(B) shows the results of measurement of the number of nodes, which is an indicator of lumen formation. Fig. 2(C) shows the results of measurement of the number of segments, which is an indicator of lumen formation.

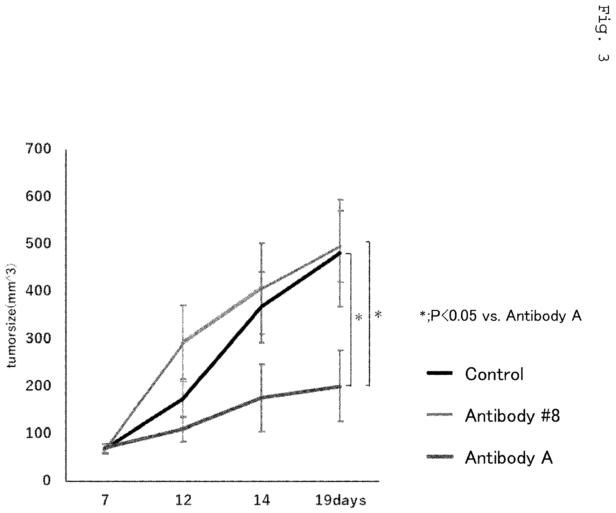

Fig. 3 shows the results of Test Example 6, which examined the effect of each antibody on tumor growth. The vertical axis represents tumor volume, and the horizontal axis represents the number of days elapsed since tumor transplant.

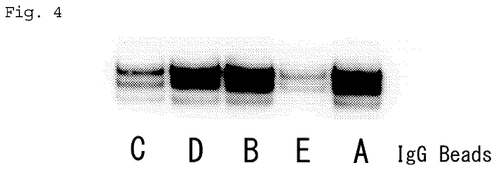

Fig. 4 shows the results of a human periostin-exon 21 recognition test with each antibody (Test Example 7). In Fig. 4, A to E denote immunoprecipitation samples of antibodies A to E. IgG denotes an immunoprecipitation sample of a negative control antibody (IgG). Beads indicate beads for antibodies in immunoprecipitation with no antibody bound (control).

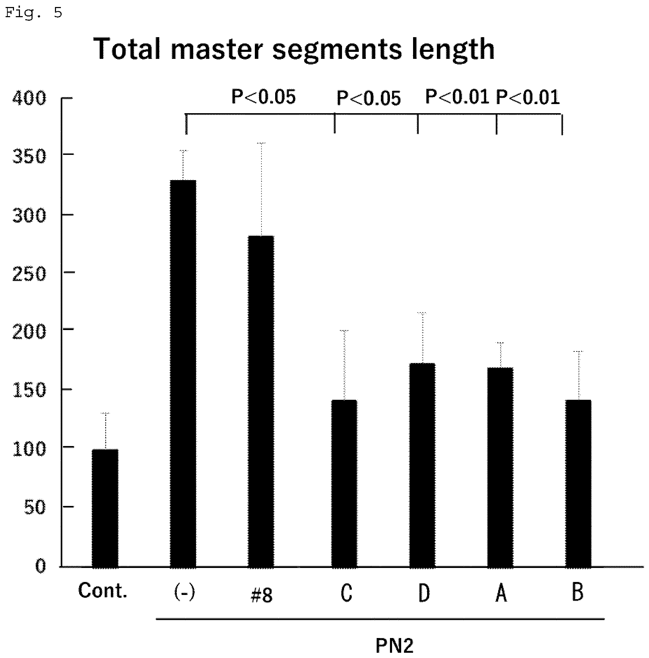

Fig. 5 shows the results of Test Example 8, which examined the effect of each antibody on vasculogenic mimicry. The vertical axis represents total master segments length, which is an indicator of lumen formation. In Fig. 5, "mPN2" denotes a sample to which mouse periostin was added. "#8," "A," "B," "C," and "D" denote samples to which antibody #8, A, B, C, and D were respectively added. "(-)" denotes a sample to which no antibody was added. "Cont." denotes a sample to which neither mouse periostin nor any antibody was added.

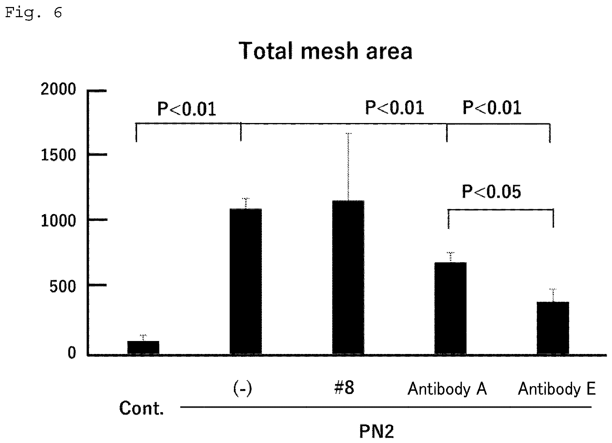

Fig. 6 shows the results of Test Example 9, which examined the effect of each antibody on vasculogenic mimicry. The vertical axis represents total mesh area (the total area of formed lumina), which is an indicator of lumen formation. In Fig. 6, "PN2" denotes a sample to which mouse periostin was added. "#8," "Antibody A," and "Antibody E" denote samples to which antibody #8, antibody A, and antibody E were respectively added. "(-)" denotes a sample to which no antibody was added. "Cont." denotes a sample to which neither mouse periostin nor any antibody was added.

### Description of Embodiments

In the present specification, the term "comprise" includes the concepts of consisting essentially of and consisting of.

### 1. Antibody or Antigen-binding Fragment Thereof

In an embodiment, the present invention relates to an antibody or antigen-binding fragment thereof bindable to a conformational epitope on human periostin, the epitope comprising the amino acid sequence A represented by SEQ ID NO: 61 and the amino acid sequence B represented by any one of SEQ ID NOs: 62 to 64 (which may be referred to as "the antibody or fragment thereof of the present invention" in the present specification). The following describes the antibody or antigen-binding fragment thereof.

The human periostin can be any human periostin that contains exon 21 (SEQ ID NO: 65) out of several existing splice variants. The human periostin is preferably a human periostin that lacks exon 17 but has exon 21, and more preferably a human periostin composed of the amino acid sequence represented by SEQ ID NO: 66 (PN-2).

The human periostin also encompasses variants that occur within the human species, as long as the structure and sequence of the conformational epitope, described below, are unchanged. In this regard, the human periostin is at least one human periostin selected from the group consisting of the following protein (a) and protein (b):
(a) a protein containing the amino acid sequence represented by SEQ ID NO: 65, and
(b) a protein containing an amino acid sequence X with at least 85% identity to the amino acid sequence represented by SEQ ID NO: 65, and containing the amino acid sequence A represented by SEQ ID NO: 61 and the amino acid sequence B represented by any one of SEQ ID NOs: 62 to 64 both within the amino acid sequence X; and preferably at least one human periostin selected from the group consisting of the following protein (c) and protein (d):
(c) a protein containing the amino acid sequence represented by SEQ ID NO: 66, and
(d) a protein containing an amino acid sequence with at least 85% identity to the amino acid sequence represented by SEQ ID NO: 66 and the amino acid sequence represented by SEQ ID NO: 65.

In proteins (b) and (d), the identity is more preferably at least 90%, still more preferably at least 95%, and yet more preferably at least 98%.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S, Altschul SF. Methods for assessing the statistical significance of molecular sequence features by using general scoring schemes, Proc Natl Acad Sci USA. 87: 2264-2268 (1990); Karlin S, Altschul SF. Applications and statistics for multiple high-scoring segments in molecular sequences, Proc Natl Acad Sci USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The "identity" of base sequences is also defined in the same manner as above.

Examples of proteins (b) and (d) include the following:
(b') a protein containing an amino acid sequence X that has the substitution, deletion, addition, or insertion (preferably substitution, and more preferably conservative substitution) of one or multiple amino acids in the amino acid sequence of SEQ ID NO: 65, and containing the amino acid sequence A represented by SEQ ID NO: 61 and the amino acid sequence B represented by any one of SEQ ID NOs: 62 to 64 both within the amino acid sequence X; and
(d') a protein containing an amino acid sequence that has the substitution, deletion, addition, or insertion (preferably substitution, and more preferably conservative substitution) of one or multiple amino acids in the amino acid sequence of SEQ ID NO: 66 and containing the amino acid sequence represented by SEQ ID NO: 65.

In proteins (b') and (d'), "multiple amino acids" means, for example, 2 to 20 amino acids, preferably 2 to 10 amino acids, more preferably 2 to 5 amino acids, and still more preferably 2 or 3 amino acids.

The phrase "conservative substitution" means the substitution of an amino acid residue with another amino acid residue having a similar side chain. For example, the substitution between amino acid residues having a basic side chain, such as lysine, arginine, or histidine, is considered to be a conservative substitution. The following substitutions between other amino acid residues are also considered to be a conservative substitution: the substitution between amino acid residues having an acidic side chain such as aspartic acid and glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

The human periostin is preferably a human periostin secreted from a human cell. The human periostin secreted from a human cell can be any human periostin that has been generated based on mRNA transcribed from the human periostin gene in a human cell and secreted extracellularly. Thus, the "human periostin secreted from a human cell" does not include recombinant human periostin that has been expressed by non-human cells (e.g., bacteria) and purified.

The human cell can be any cell capable of secreting human periostin. Examples of human cells include cancer cells. The cancer from which cancer cells are originated is, for example, brain tumor, leukemia, osteosarcoma, breast cancer, large intestine cancer, malignant melanoma, bone cancer, stomach cancer, lung cancer, liver cancer, kidney cancer, pancreas cancer, gallbladder cancer, skin cancer, uterus cancer, ovarian cancer, rectal cancer, colon cancer, fallopian tube cancer, esophageal cancer, small intestine cancer, thyroid cancer, parathyroid cancer, adrenal cancer, prostate cancer, bladder cancer, or malignant lymphoma, preferably breast cancer, large intestine cancer, lung cancer, or malignant melanoma, and particularly preferably breast cancer.

The antibody or a fragment thereof of the present invention is bindable to a conformational epitope on human periostin. The conformational epitope on human periostin is not particularly limited as long as the epitope has a three-dimensional structure containing the amino acid sequence A represented by SEQ ID NO: 61 and the amino acid sequence B represented by any one of SEQ ID NOs: 62 to 64. In other words, the term "bindable" means that the antibody or fragment thereof of the present invention can recognize a three-dimensional structure on human periostin or bind to the three-dimensional structure. The antibody or fragment thereof of the present invention is preferably specifically bindable to a conformational epitope on human periostin. The amino acid sequence B is preferably SEQ ID NO: 62.

In a preferable embodiment, the antibody or fragment thereof of the present invention may be an antibody or fragment thereof comprising
heavy-chain CDR 1 comprising the amino acid sequence represented by SEQ ID NO: 1 and/or 2, SEQ ID NO: 13 and/or 14, SEQ ID NO: 25 and/or 26, SEQ ID NO: 37 and/or 38, or SEQ ID NO: 49 and/or 50,
heavy-chain CDR 2 comprising the amino acid sequence represented by SEQ ID NO: 3 and/or 4, SEQ ID NO: 15 and/or 16, SEQ ID NO: 27 and/or 28, SEQ ID NO: 39 and/or 40, or SEQ ID NO: 51 and/or 52,
heavy-chain CDR 3 comprising the amino acid sequence represented by SEQ ID NO: 5 and/or 6, SEQ ID NO: 17 and/or 18, SEQ ID NO: 29 and/or 30, SEQ ID NO: 41 and/or 42, or SEQ ID NO: 53 and/or 54,
light-chain CDR 1 comprising the amino acid sequence represented by SEQ ID NO: 7 and/or 8, SEQ ID NO: 19 and/or 20, SEQ ID NO: 31 and/or 32, SEQ ID NO: 43 and/or 44, or SEQ ID NO: 55 and/or 56,
light-chain CDR 2 comprising the amino acid sequence represented by SEQ ID NO: 9 and/or 10, SEQ ID NO: 21 and/or 22, SEQ ID NO: 33 and/or 34, SEQ ID NO: 45 and/or 46, or SEQ ID NO: 57 and/or 58, and
light-chain CDR 3 comprising the amino acid sequence represented by SEQ ID NO: 11 and/or 12, SEQ ID NO: 23 and/or 24, SEQ ID NO: 35 and/or 36, SEQ ID NO: 47 and/or 48, or SEQ ID NO: 59 and/or 60.

In the preferable embodiment above, the amino acid sequences represented by an odd sequence number are CDR sequences defined by the Kabat numbering scheme, and the amino acid sequences represented by an even sequence number are CDR sequences defined by the IMGT numbering scheme.

In a more preferable embodiment, the antibody or fragment thereof of the present invention may be an antibody or antigen-binding fragment comprising the following (A), (B), (C), (D), or (E) :
(A)
   heavy-chain CDR 1 comprising the amino acid sequence represented by SEQ ID NO: 1 and/or 2,
   heavy-chain CDR 2 comprising the amino acid sequence represented by SEQ ID NO: 3 and/or 4,
   heavy-chain CDR 3 comprising the amino acid sequence represented by SEQ ID NO: 5 and/or 6,
   light-chain CDR 1 comprising the amino acid sequence represented by SEQ ID NO: 7 and/or 8,
   light-chain CDR 2 comprising the amino acid sequence represented by SEQ ID NO: 9 and/or 10, and
   light-chain CDR 3 comprising the amino acid sequence represented by SEQ ID NO: 11 and/or 12;
(B)
   heavy-chain CDR 1 comprising the amino acid sequence represented by SEQ ID NO: 13 and/or 14,
   heavy-chain CDR 2 comprising the amino acid sequence represented by SEQ ID NO: 15 and/or 16,
   heavy-chain CDR 3 comprising the amino acid sequence represented by SEQ ID NO: 17 and/or 18,
   light-chain CDR 1 comprising the amino acid sequence represented by SEQ ID NO: 19 and/or 20,
   light-chain CDR 2 comprising the amino acid sequence represented by SEQ ID NO: 21 and/or 22, and
   light-chain CDR 3 comprising the amino acid sequence represented by SEQ ID NO: 23 and/or 24;
(C)
   heavy-chain CDR 1 comprising the amino acid sequence represented by SEQ ID NO: 25 and/or 26,
   heavy-chain CDR 2 comprising the amino acid sequence represented by SEQ ID NO: 27 and/or 28,
   heavy-chain CDR 3 comprising the amino acid sequence represented by SEQ ID NO: 29 and/or 30,
   light-chain CDR 1 comprising the amino acid sequence represented by SEQ ID NO: 31 and/or 32,
   light-chain CDR 2 comprising the amino acid sequence represented by SEQ ID NO: 33 and/or 34, and
   light-chain CDR 3 comprising the amino acid sequence represented by SEQ ID NO: 35 and/or 36;
(D)
   heavy-chain CDR 1 comprising the amino acid sequence represented by SEQ ID NO: 37 and/or 38,
   heavy-chain CDR 2 comprising the amino acid sequence represented by SEQ ID NO: 39 and/or 40,
   heavy-chain CDR 3 comprising the amino acid sequence represented by SEQ ID NO: 41 and/or 42,
   light-chain CDR 1 comprising the amino acid sequence represented by SEQ ID NO: 43 and/or 44,
   light-chain CDR 2 comprising the amino acid sequence represented by SEQ ID NO: 45 and/or 46, and
   light-chain CDR 3 comprising the amino acid sequence represented by SEQ ID NO: 47 and/or 48; or
(E)
   heavy-chain CDR 1 comprising the amino acid sequence represented by SEQ ID NO: 49 and/or 50,
   heavy-chain CDR 2 comprising the amino acid sequence represented by SEQ ID NO: 51 and/or 52,
   heavy-chain CDR 3 comprising the amino acid sequence represented by SEQ ID NO: 53 and/or 54,
   light-chain CDR 1 comprising the amino acid sequence represented by SEQ ID NO: 55 and/or 56,
   light-chain CDR 2 comprising the amino acid sequence represented by SEQ ID NO: 57 and/or 58, and
   light-chain CDR 3 comprising the amino acid sequence represented by SEQ ID NO: 59 and/or 60.

Of the antibodies or antigen-binding fragments thereof described in (A) to (E), the antibody or fragment thereof of the present invention is more preferably the antibody or antigen-binding fragment thereof described in (A) or (B), and particularly preferably the antibody or antigen-binding fragment thereof described in (A). In another embodiment, the antibody or fragment thereof of the present invention is more preferably the antibody or antigen-binding fragment thereof described in (A) or (E), and particularly preferably the antibody or antigen-binding fragment thereof described in (E).

In a more preferable embodiment, the antibody or fragment thereof of the present invention may be an antibody or antigen-binding fragment thereof comprising
(A1)
   heavy-chain CDR 1 comprising the amino acid sequence represented by SEQ ID NO: 1,
   heavy-chain CDR 2 comprising the amino acid sequence represented by SEQ ID NO: 3,
   heavy-chain CDR 3 comprising the amino acid sequence represented by SEQ ID NO: 5,
   light-chain CDR 1 comprising the amino acid sequence represented by SEQ ID NO: 7,
   light-chain CDR 2 comprising the amino acid sequence represented by SEQ ID NO: 9, and
   light-chain CDR 3 comprising the amino acid sequence represented by SEQ ID NO: 11; or
(A2)
   heavy-chain CDR 1 comprising the amino acid sequence represented by SEQ ID NO: 2,
   heavy-chain CDR 2 comprising the amino acid sequence represented by SEQ ID NO: 4,
   heavy-chain CDR 3 comprising the amino acid sequence represented by SEQ ID NO: 6,
   light-chain CDR 1 comprising the amino acid sequence represented by SEQ ID NO: 8,
   light-chain CDR 2 comprising the amino acid sequence represented by SEQ ID NO: 10, and
   light-chain CDR 3 comprising the amino acid sequence represented by SEQ ID NO: 12; or
(B1)
   heavy-chain CDR 1 comprising the amino acid sequence represented by SEQ ID NO: 13,
   heavy-chain CDR 2 comprising the amino acid sequence represented by SEQ ID NO: 15,
   heavy-chain CDR 3 comprising the amino acid sequence represented by SEQ ID NO: 17,
   light-chain CDR 1 comprising the amino acid sequence represented by SEQ ID NO: 19,
   light-chain CDR 2 comprising the amino acid sequence represented by SEQ ID NO: 21, and
   light-chain CDR 3 comprising the amino acid sequence represented by SEQ ID NO: 23; or
(B2)
   heavy-chain CDR 1 comprising the amino acid sequence represented by SEQ ID NO: 14,
   heavy-chain CDR 2 comprising the amino acid sequence represented by SEQ ID NO: 16,
   heavy-chain CDR 3 comprising the amino acid sequence represented by SEQ ID NO: 18,
   light-chain CDR 1 comprising the amino acid sequence represented by SEQ ID NO: 20,
   light-chain CDR 2 comprising the amino acid sequence represented by SEQ ID NO: 22, and
   light-chain CDR 3 comprising the amino acid sequence represented by SEQ ID NO: 24; or
(C1)
   heavy-chain CDR 1 comprising the amino acid sequence represented by SEQ ID NO: 25,
   heavy-chain CDR 2 comprising the amino acid sequence represented by SEQ ID NO: 27,
   heavy-chain CDR 3 comprising the amino acid sequence represented by SEQ ID NO: 29,
   light-chain CDR 1 comprising the amino acid sequence represented by SEQ ID NO: 31,
   light-chain CDR 2 comprising the amino acid sequence represented by SEQ ID NO: 33, and
   light-chain CDR 3 comprising the amino acid sequence represented by SEQ ID NO: 35; or
(C2)
   heavy-chain CDR 1 comprising the amino acid sequence represented by SEQ ID NO: 26,
   heavy-chain CDR 2 comprising the amino acid sequence represented by SEQ ID NO: 28,
   heavy-chain CDR 3 comprising the amino acid sequence represented by SEQ ID NO: 30,
   light-chain CDR 1 comprising the amino acid sequence represented by SEQ ID NO: 32,
   light-chain CDR 2 comprising the amino acid sequence represented by SEQ ID NO: 34, and
   light-chain CDR 3 comprising the amino acid sequence represented by SEQ ID NO: 36; or
(D1)
   heavy-chain CDR 1 comprising the amino acid sequence represented by SEQ ID NO: 37 and/or 38,
   heavy-chain CDR 2 comprising the amino acid sequence represented by SEQ ID NO: 39 and/or 40,
   heavy-chain CDR 3 comprising the amino acid sequence represented by SEQ ID NO: 41 and/or 42,
   light-chain CDR 1 comprising the amino acid sequence represented by SEQ ID NO: 43 and/or 44,
   light-chain CDR 2 comprising the amino acid sequence represented by SEQ ID NO: 45 and/or 46, and
   light-chain CDR 3 comprising the amino acid sequence represented by SEQ ID NO: 47 and/or 48; or
(D2)
   heavy-chain CDR 1 comprising the amino acid sequence represented by SEQ ID NO: 38,
   heavy-chain CDR 2 comprising the amino acid sequence represented by SEQ ID NO: 40,
   heavy-chain CDR 3 comprising the amino acid sequence represented by SEQ ID NO: 42,
   light-chain CDR 1 comprising the amino acid sequence represented by SEQ ID NO: 44,
   light-chain CDR 2 comprising the amino acid sequence represented by SEQ ID NO: 46, and
   light-chain CDR 3 comprising the amino acid sequence represented by SEQ ID NO: 48; or
(E1)
   heavy-chain CDR 1 comprising the amino acid sequence represented by SEQ ID NO: 49,
   heavy-chain CDR 2 comprising the amino acid sequence represented by SEQ ID NO: 51,
   heavy-chain CDR 3 comprising the amino acid sequence represented by SEQ ID NO: 53,
   light-chain CDR 1 comprising the amino acid sequence represented by SEQ ID NO: 55,
   light-chain CDR 2 comprising the amino acid sequence represented by SEQ ID NO: 57, and
   light-chain CDR 3 comprising the amino acid sequence represented by SEQ ID NO: 59; or
(E2)
   heavy-chain CDR 1 comprising the amino acid sequence represented by SEQ ID NO: 50,
   heavy-chain CDR 2 comprising the amino acid sequence represented by SEQ ID NO: 52,
   heavy-chain CDR 3 comprising the amino acid sequence represented by SEQ ID NO: 54,
   light-chain CDR 1 comprising the amino acid sequence represented by SEQ ID NO: 56,
   light-chain CDR 2 comprising the amino acid sequence represented by SEQ ID NO: 58, and
   light-chain CDR 3 comprising the amino acid sequence represented by SEQ ID NO: 60.

Of the antibodies or antigen-binding fragments thereof described in (A1) to (E2), the antibody or fragment thereof of the present invention is more preferably the antibody or antigen-binding fragment thereof described in (A1), (A2), (B1), or (B2), and particularly preferably the antibody or antigen-binding fragment thereof described in (A1) or (A2). In another embodiment, the antibody or fragment thereof of the present invention is more preferably the antibody or antigen-binding fragment thereof described in (A1), (A2), (E1), or (E2), and particularly preferably the antibody or antigen-binding fragment thereof described in (E1) or (E2).

The antibody or antigen-binding fragment thereof described in (A) preferably contains a heavy-chain variable region containing the amino acid sequence AH1 represented by SEQ ID NO: 67, an amino acid sequence AH2 with at least 85% (preferably at least 90%, more preferably at least 95%, still more preferably at least 98%, and yet more preferably at least 99%) identity to the amino acid sequence AH1, or the amino acid sequence AH3, which is a humanized sequence of the amino acid sequence AH1 and/or a light-chain variable region containing the amino acid sequence AL1 represented by SEQ ID NO: 68, an amino acid sequence AL2 with at least 85% (preferably at least 90%, more preferably at least 95%, still more preferably at least 98%, and yet more preferably at least 99%) identity to the amino acid sequence AL1, or the amino acid sequence AL3, which is a humanized sequence of the amino acid sequence AL1.

The antibody or antigen-binding fragment thereof described in (B) preferably contains a heavy-chain variable region containing the amino acid sequence BH1 represented by SEQ ID NO: 69, an amino acid sequence BH2 with at least 85% (preferably at least 90%, more preferably at least 95%, still more preferably at least 98%, and yet more preferably at least 99%) identity to the amino acid sequence BH1, or the amino acid sequence BH3, which is a humanized sequence of the amino acid sequence BH1 and/or a light-chain variable region containing the amino acid sequence BL1 represented by SEQ ID NO: 70, an amino acid sequence BL2 with at least 85% (preferably at least 90%, more preferably at least 95%, still more preferably at least 98%, and yet more preferably at least 99%) identity to the amino acid sequence BL1, or the amino acid sequence BL3, which is a humanized sequence of the amino acid sequence BL1.

The antibody or antigen-binding fragment thereof described in (C) preferably contains a heavy-chain variable region containing the amino acid sequence CH1 represented by SEQ ID NO: 71, an amino acid sequence CH2 with at least 85% (preferably at least 90%, more preferably at least 95%, still more preferably at least 98%, and yet more preferably at least 99%) identity to the amino acid sequence CH1, or the amino acid sequence CH3, which is a humanized sequence of the amino acid sequence CH1 and/or a light-chain variable region containing the amino acid sequence CL1 represented by SEQ ID NO: 72, the amino acid sequence CL2 with at least 85% (preferably at least 90%, more preferably at least 95%, still more preferably at least 98%, and yet more preferably at least 99%) identity to the amino acid sequence CL1, or the amino acid sequence CL3, which is a humanized sequence of the amino acid sequence CL1.

The antibody or antigen-binding fragment thereof described in (D) preferably contains a heavy-chain variable region containing the amino acid sequence DH1 represented by SEQ ID NO: 73, an amino acid sequence DH2 with at least 85% (preferably at least 90%, more preferably at least 95%, still more preferably at least 98%, and yet more preferably at least 99%) identity to the amino acid sequence DH1, or the amino acid sequence DH3, which is a humanized sequence of the amino acid sequence DH1 and/or a light-chain variable region containing the amino acid sequence DL1 represented by SEQ ID NO: 74, an amino acid sequence DL2 with at least 85% (preferably at least 90%, more preferably at least 95%, still more preferably at least 98%, and yet more preferably at least 99%) identity to the amino acid sequence DL1, or the amino acid sequence DL3, which is a humanized sequence of the amino acid sequence DL1.

The antibody or antigen-binding fragment thereof described in (E) preferably contains a heavy-chain variable region containing the amino acid sequence EH1 represented by SEQ ID NO: 75, an amino acid sequence EH2 with at least 85% (preferably at least 90%, more preferably at least 95%, still more preferably at least 98%, and yet more preferably at least 99%) identity to the amino acid sequence EH1, or the amino acid sequence EH3, which is a humanized sequence of the amino acid sequence EH1 and/or a light-chain variable region containing the amino acid sequence EL1 represented by SEQ ID NO: 76, an amino acid sequence EL2 with at least 85% (preferably at least 90%, more preferably at least 95%, still more preferably at least 98%, and yet more preferably at least 99%) identity to the amino acid sequence EL1, or the amino acid sequence EL3, which is a humanized sequence of the amino acid sequence EL1.

The term "antibody" is used in the meaning including monoclonal antibodies and polyclonal antibodies. The antibody may be any isotype, such as IgG (e.g., IgG1, IgG2, IgG3, and IgG4), IgA (e.g., IgA1 and IgA2), IgD, IgE, or IgM. The antibody may be a human antibody or a non-human antibody. Examples of non-human antibodies include, but are not limited to, mouse antibodies, rat antibodies, rabbit antibodies, monkey antibodies, and chimpanzee antibodies. The antibody may be a chimeric antibody, such as mouse-human chimeric antibodies. The antibody may be a partially or fully humanized antibody. The antibody of the present invention is preferably a monoclonal antibody.

In the present specification, the antigen-binding fragment of the antibody can be any fragment that contains heavy-chain CDRs 1 to 3 and light-chain CDRs 1 to 3, such as Fab, F(ab')₂, minibody, scFv-Fc, Fv, scFv, diabody, triabody, or tetrabody.

"Fab" contains a fragment of a heavy chain containing the heavy-chain variable region and CH1 in the heavy-chain constant region and a light chain containing the light-chain variable region and the light-chain constant region (CL), and the heavy-chain variable region and the light-chain variable region are aggregated by non-covalent, intermolecular interaction described above, or bound to each other through the disulfide bond. In Fab, CH1 and CL may be disulfide-bonded by the thiol groups of the respective cysteine residues.

"F(ab')₂" refers to the structure that contains a pair of Fabs in which CH1 of one Fab is disulfide-bonded to CH1 of another Fab by thiol groups of the respective cysteine residues.

"Minibody" refers to the structure in which two fragments each containing CH3 bound to a heavy-chain variable region constituting scFV are aggregated between CH3-CH3 by non-covalent, intermolecular interaction.

"scFv-Fc" refers to the structure in which two antibody fragments each containing scFv, CH2, and CH3 are aggregated between CH3-CH3 by non-covalent, intermolecular interaction, as with the minibody, and the fragments are disulfide-bonded through thiol groups of the cysteine residues contained in respective CH3.

"Fv" is considered to be the smallest structure unit of an antibody and has the heavy-chain variable region and the light-chain variable region that are aggregated by non-covalent, intermolecular interaction. In Fv, a thiol group of the cysteine residue present in the heavy-chain variable region and a thiol group of the cysteine residue present in the light-chain variable region may be disulfide bonded.

"scFv" has the structure in which the C-terminus of the heavy-chain variable region and the N-terminus of the light-chain variable region are bound through a linker or the structure in which the N-terminus of the heavy-chain variable region and the C-terminus of the light-chain variable region are bound through a linker, and is also referred to as a "single-chain antibody."

The "diabody," "triabody," and "tetrabody" respectively refer to a dimer, a trimer, and a tetramer formed by scFv described above and are each an aggregated and structurally stabilized structure, for example, due to non-covalent, intermolecular interaction between the variable regions, as with Fv.

The antibody or fragment thereof of the present invention may be bound or fused to another peptide, oligopeptide, or protein. Examples of such other peptides, oligopeptides, or proteins include albumin (e.g., serum albumin), protein tags (e.g., biotin, His tag, FLAG tag, Halo tag, MBP tag, HA tag, Myc tag, V5 tag, and PA tag), fluorescent proteins (e.g., GFP, Azami-Green, ZsGreen, GFP2, HyPer, Sirius, BFP, CFP, Turquoise, Cyan, TFP1, YFP, Venus, ZsYellow, Banana, KusabiraOrange, RFP, DsRed, AsRed, Strawberry, Jred, KillerRed, Cherry, HcRed, and mPlum), photoproteins (e.g., luciferase, β-galactosidase, chloramphenicol acetyltransferase, and β-glucuronidase), secretory signal sequences (e.g., Igκ signal sequence), protease recognition sequences (e.g., TEV protease recognition sequence), expression-enhancing sequences, solubilization sequences, multimeric domains (e.g., cartilage oligomer matrix protein domain, leucine zipper domain, collagen-like domain, cholera toxin B subunit domain, tetrabrachion coiled core domain, reovirus σ1 protein domain, and hepatitis delta antigen domain). If the antibody or fragment thereof of the present invention is bound or fused to a multimeric domain, the multimeric domain may further be bound or fused to an antibody or antigen-binding fragment thereof that is of the same type as or different type from the antibody or fragment thereof of the present invention to form a multimer (homomultimer or heteromultimer).

The antibody or fragment thereof of the present invention may be chemically modified to the extent that the binding properties to human periostin are not significantly impaired.

The antibody or fragment thereof of the present invention may have a carboxyl group (-COOH), carboxylate (-COO⁻), an amide group (-CONH₂), or an ester group (-COOQ) at the C-terminus. Q in the ester group may be, for example, a C₁₋₆ alkyl group, such as methyl, ethyl, n-propyl, isopropyl, or n-butyl; a C₃₋₈ cycloalkyl group, such as cyclopentyl or cyclohexyl; a C₆₋₁₂ aryl group, such as phenyl or α-naphthyl; a phenyl-C₁₋₂ alkyl group, such as benzyl or phenethyl; a C₇₋₁₄ aralkyl group, such as an α-naphthyl-C₁₋₂ alkyl group, including α-naphthylmethyl; or a pivaloyloxymethyl group.

The antibody or fragment thereof of the present invention may have an amidated or esterified carboxyl group (or carboxylate) at a site other than the C-terminus. The ester in this case may be, for example, the esters of the C-terminus described above.

The antibody or fragment thereof of the present invention includes those having the amino group of the N-terminal amino acid residue protected by a protective group (e.g., a C₁₋₆ acyl group, including a C₁₋₆ alkanoyl, such as a formyl group and an acetyl group); those having the N-terminal glutamine residue (which are formed due to cleavage in vivo) pyroglutamated; those having a substituent (e.g., -OH, -SH, an amino group, an imidazole group, an indole group, and a guanidino group) on the side chain of an amino acid in the molecule protected by an appropriate protective group (e.g., a C₁₋₆ acyl group, including a C₁₋₆ alkanoyl group, such as a formyl group and an acetyl group); and complex proteins, such as glycoproteins to which a carbohydrate chain is attached.

### 2. Method for Producing Antibody or Antigen-binding Fragment Thereof

The antibody or fragment thereof of the present invention can be produced according to various methods.

The antibody or fragment thereof of the present invention can be produced, for example, according to a method including selecting an antibody that is bindable to human periostin secreted from a human breast cancer cell from among antibodies produced by a hybridoma obtained from an animal immunized with at least one protein or peptide selected from the group consisting of:
(e) a protein or peptide containing the amino acid sequence represented by SEQ ID NO: 65, and
(f) a protein or peptide containing an amino acid sequence X with at least 85% identity to the amino acid sequence represented by SEQ ID NO: 65, and containing the amino acid sequence A represented by SEQ ID NO: 61 and the amino acid sequence B represented by any one of SEQ ID NOs: 62 to 64 both within the amino acid sequence X.

From this viewpoint, in an embodiment of the present invention, the antibody or fragment thereof of the present invention may be an antibody obtained according to a method including selecting an antibody that is bindable to human periostin secreted from a human breast cancer cell from among antibodies produced by a hybridoma obtained from an animal immunized with at least one protein or peptide selected from the group consisting of:
(e) a protein or peptide containing the amino acid sequence represented by SEQ ID NO: 65, and
(f) a protein or peptide containing an amino acid sequence X with at least 85% identity to the amino acid sequence represented by SEQ ID NO: 65 and containing the amino acid sequence A represented by SEQ ID NO: 61 and the amino acid sequence B represented by any one of SEQ ID NOs: 62 to 64 both within the amino acid sequence X.

From this viewpoint, in an embodiment of the present invention, the antibody or fragment thereof of the present invention may be an antibody that is produced by a hybridoma obtained from an animal immunized with at least one protein or peptide selected from the group consisting of the following (e) and (f), and that is bindable to human periostin secreted from a human breast cancer cell:
(e) a protein or peptide containing the amino acid sequence represented by SEQ ID NO: 65, and
(f) a protein or peptide containing an amino acid sequence X with at least 85% identity to the amino acid sequence represented by SEQ ID NO: 65, and containing the amino acid sequence A represented by SEQ ID NO: 61 and the amino acid sequence B represented by any one of SEQ ID NOs: 62 to 64 both within the amino acid sequence X.

The proteins and peptides described in (e) and (f) are those as described in (a) and (b).

The animal to be immunized can be any animal from which a hybridoma can be prepared, and is preferably an animal with an autoimmune disease (in the case of mice, for example, BXSB mice). Immunization and preparation of a hybridoma can be performed according to a known method (e.g., Current protocols in Molecular Biology edit. Ausubel et al. (1987) publish. John Wiley and Sons. Sections 11.4 to 11.11). The evaluation method for the method for selecting antibodies bindable to human periostin secreted from human breast cancer cells is not particularly limited as long as the method uses an antigen-antibody reaction by using human periostin secreted from human breast cancer cells. For example, the method described in Test Example 2, described below, may be used.

In the present specification, the binding of the antibody or a fragment thereof to the target (e.g., human periostin secreted from human breast cancer cells) can be measured according to the surface plasmon resonance (SRP) method. In an embodiment of the present invention, the dissociation constant (K_{D} value) between the antibody or fragment thereof of the present invention and their target (e.g., human periostin secreted from human breast cancer cells) is, for example, 9×10⁻⁶ M or less, preferably 9×10⁻⁸ M or less, and more preferably 9×10⁻¹⁰ M or less. The lower limit of the dissociation constant is not particularly limited and may be, for example, 1×10⁻¹⁴ M, 1×10⁻¹³ M, 1×10⁻¹² M, or 1×10⁻¹¹ M.

In another embodiment, the antibody or fragment thereof of the present invention may be produced, for example, according to a method including culturing a host transformed with a polynucleotide containing the coding sequence of the antibody or fragment thereof of the present invention (the polynucleotide of the invention) and collecting a fraction containing the antibody of the present invention.

The polynucleotide of the present invention can be any polynucleotide that contains the coding sequence of the antibody or fragment thereof of the present invention. Preferably, the polynucleotide of the present invention contains the coding sequence in such a manner that the antibody or fragment thereof of the present invention can be expressed. The polynucleotide of the present invention may contain other sequences in addition to the coding sequence. Other sequences include a secretory signal peptide coding sequence, a promoter sequence, an enhancer sequence, a repressor sequence, an insulator sequence, origin of replication, and a coding sequence of a drug-resistant gene, which are arranged adjacent to the coding sequence of the antibody of the present invention. The polynucleotide of the present invention may be a linear polynucleotide or a cyclic polynucleotide (e.g., a vector).

Specific examples of the polynucleotide of the present invention include (I) a polynucleotide containing a base sequence encoding at least one member selected from the group consisting of the heavy chain, the heavy-chain variable region, and heavy-chain CDRs 1-3 of the antibody of the present invention, (II) a polynucleotide containing a base sequence encoding at least one member selected from the group consisting of the light chain, the light-chain variable region, and light-chain CDRs 1-3 of the antibody of the present invention, and (III) a polynucleotide containing a nucleic acid containing a base sequence encoding at least one member selected from the group consisting of the heavy chain, the heavy-chain variable region, and heavy-chain CDRs 1-3 of the antibody of the present invention; and a base sequence encoding at least one member selected from the group consisting of the light chain, the light-chain variable region, and light-chain CDRs 1-3 of the antibody of the present invention.

The host is not particularly limited, and examples of hosts include insect cells, eukaryotic cells, and mammal cells. In particular, from the viewpoint of efficiently expressing an antibody, mammal cells such as HEK cells, CHO cells, NS0 cells, and SP2/O cells are preferable.

The methods for transformation, culture, and collection are not particularly limited, and methods known in antibody production can be used.

After collection, the antibody of the present invention may optionally be purified. Purification can be performed according to methods known in antibody production, such as chromatography and dialysis.

Polynucleotides such as DNA and RNA may be chemically modified as in the examples described below. To prevent degradation by hydrolases, such as nucleases, the phosphate residue (phosphate) of each nucleotide can be replaced with a chemically modified phosphate residue, such as phosphorothioate (PS), methylphosphonate, or phosphorodithionate. The hydroxyl group at position 2 of the ribose of each ribonucleotide may be replaced with -OR (R represents, for example, -CH₃, -CH₂CH₂OCH₃, -CH₂CH₂NHC(NH)NH₂, -CH₂CONHCH₃, or -CH₂CH₂CN). Additionally, the base moiety (pyrimidine, purine) may be chemically modified by, for example, introduction of a methyl group or a cationic functional group into position 5 of the pyrimidine base, or replacement of the carbonyl group at position 2 with thiocarbonyl. Additionally, the polynucleotide of the present invention also includes, but is not limited to, those in which the phosphate moiety or hydroxyl moiety is modified with biotin, an amino group, a lower alkyl amine group, an acetyl group, or the like.

### 2-1. Preparation of Humanized Antibody

If the antibody of the present invention is obtained as a humanized antibody, the antibody can be prepared based on the following information, and optionally in accordance with or pursuant to other known information.

Immunoglobulin G (simply "IgG" below) is composed of two light polypeptide chains with a molecular weight of about 23000 ("light chain" below) and two heavy polypeptide chains with a molecular weight of about 50000 ("heavy chain" below). The heavy chains and light chains both have a repeating structure of the region of a conserved amino acid sequence of about 110 residues, and these are the basic unit of the three-dimensional structure of IgG ("domain" below). The heavy chains and light chains consist of 4 and 2 successive domains, respectively. The amino terminal domains of the heavy and light chains are more variable in amino acid sequence between antibody molecules than other domains, and thus the amino terminal domains are referred to as "variable domains" ("V domain" below). At each amino terminus of IgG, the heavy-chain V domain and the light-chain V domain complementarily associate to form a variable region. The remaining domains collectively form a constant region. The sequence of the constant region is divergent between animal species. For example, the constant region of mouse IgG differs from the constant region of human IgG, and thus mouse IgG is recognized as a foreign matter by the human immune system, resulting in a Human Anti-Mouse Antibody ("HAMA" below) response (Schroff R. W. et al. Cancer Res. (1985) 45, 879-85). Mouse antibodies thus cannot be repeatedly administered to a human. In order to administer such antibodies to a human, the antibody molecules must be modified to prevent the HAMA response without losing the specificity of the antibodies.

According to the results of X-ray crystallography, these domains are generally in the form of a long cylindrical structure made up of a two-layered anti-parallel β sheets composed of 3 to 5 β chains. In the variable regions, three loops are assembled to form an antigen-binding site in each of the V domains of the heavy and light chains. These loops are referred to as "complementarity determining regions" ("CDRs" below), which are most variable in amino acid sequence. The parts of the variable region other than the CDRs play a role in maintaining the structure of the CDRs and are referred to as "frameworks." Kabatt et al. collected a large number of the primary sequences of heavy-chain and light-chain variable regions, and created a table in which the primary sequences are classified into CDRs and frameworks on the basis of sequence conservation (Kabat et al. Sequences of Proteins of Immunological Interest, 5th edition, NIH publication, No. 91-3242, E.A.).

The frameworks were further classified into a plurality of subgroups based on shared amino acid sequence patterns. The existence of corresponding frameworks between humans and mice was also found. Such studies on the structural features of IgG led to the development of the methods for producing humanized antibodies described below. At an early stage of the studies, chimeric antibodies having a variable region from a mouse antibody fused to a constant region from a human antibody were proposed (Morrison S. L. et al. Proc Natl Acad Sci USA. (1984) 81, 6851-5). However, since such chimeric antibodies still contain many non-human amino acid residues, the antibodies may induce the HAMA response, especially when they are administered for a long period of time (Begent et al., Br. J. Cancer, (1990) 62, 487).

To further reduce amino acid residues derived from a non-human mammal that may induce the HAMA response in humans, a method that incorporates only CDRs into an antibody of human origin (Peter T. et al. Nature, (1986) 321, 522-5) was proposed. In general, however, grafting only CDRs was insufficient to maintain immunoglobulin activity against an antigen. In 1987, Chothia et al. used the data of X-ray crystallography to find the following: (a) the amino acid sequences of the CDRs contain the sites that directly bind to an antigen and the sites that maintain the structures of the CDRs, and possible three-dimensional structures of the CDRs are classified into several typical patterns (canonical structures); and (b) the canonical structure classes are determined by not only the CDRs but also the types of the amino acids located at specific positions in the frameworks (Chothia C. et al. J. Mol. Biol. (1987) 196, 901-17). These findings suggested that in CDR grafting, some amino acid residues in the frameworks, in addition to the CDR sequences, should also be grafted into a human antibody (JPH04-502408A).

In general, an antibody derived from a non-human mammal having CDRs to be grafted to a human antibody is defined as "donor," and the human antibody into which the CDRs are to be grafted is defined as "acceptor." In CDR grafting, the structure of the CDRs should be conserved as much as possible to maintain the activity of the immunoglobulin molecule. To achieve this, two points to note are: (a) from which subgroup the acceptor should be selected, and (b) which amino acid residues should be selected from the donor frameworks.

Queen et al. proposed a method for designing a humanized antibody by grafting amino acid residues from donor frameworks together with the CDR sequences into an acceptor when the amino acid residues from the donor frameworks satisfy at least one of the following criteria (JPH04-502408A):
(a) the amino acids to be substituted are rare for their positions in the acceptor framework region, and the corresponding amino acids from the donor are common for their positions in the acceptor framework region;
(b) the amino acids are immediately adjacent to one of the CDRs; and
(c) the amino acids are predicted to have a side chain atom within about 3Å (angstrom) of the CDRs in a three-dimensional immunoglobulin model and to be capable of interacting with an antigen or with the CDRs of the humanized antibody.

### 2-2. Production of Human Antibody

If the antibody of the present invention is to be obtained as a human antibody, the antibody can be produced based on the following information, and in accordance with or pursuant to other known information, as necessary.

The term "human antibody" or "human immunoglobulin" in the present invention refers to an immunoglobulin in which its constituent regions including the heavy-chain variable regions (VH), the heavy-chain constant regions (CH), the light-chain variable regions (VL), and the light-chain constant regions (CL) are all derived from a gene encoding a human immunoglobulin. In other words, the term refers to an antibody in which the heavy chains are derived from a human immunoglobulin heavy-chain gene and the light chains are derived from a human immunoglobulin light-chain gene.

A human antibody can be produced in a manner similar to the method for producing a monoclonal antibody according to a commonly used method, for example, by incorporating at least a human immunoglobulin gene into the locus of a gene of a non-human mammal (e.g., a mouse) to create a transgenic animal, and subjecting the transgenic animal to immune sensitization with an antigen. Transgenic mice producing human antibodies can be created, for example, according to the methods previously reported (Mendez M. J. et al. Nature Genetics (1997) 15, 146-56; Green L. L. et al. Nature Genetics (1994) 7, 13-21; JPH04-504365A; WO94/25585; Nikkei Science, June, pp. 40-50, 1995; Nils Lonberg et al. Nature (1994) 368, 856-9; and JPH06-500233A).

### 3. Cell

In an embodiment, the present invention relates to a cell containing the polynucleotide of the present invention.

The cell includes, for example, cells transfected with the polynucleotide of the present invention

The cell is preferably an isolated cell. The cell may be a cultured cell, a primary cultured cell, or a subcultured cell. The cell may be a strain cell. The cell may be an iPS cell.

Examples of the cell include *Escherichia coli* such as *Escherichia coli* K12; bacteria of the genus *Bacillus,* such as *Bacillus subtilis* MI114; yeast, such as *Saccharomyces cerevisiae* AH22; Sf cell lines derived from *Spodoptera frugiperda* or HighFive cell lines derived from *Trichoplusia ni;* insect cells, such as olfactory nerve cells; and animal cells. Animal cells are preferably cultured cells of mammalian origin, specifically such as COS7 cells, CHO cells, HEK293 cells, HEK293FT cells, HeLa cells, PC12 cells, N1E-115 cells, and SH-SY5Y cells.

### 4. Conjugate

In an embodiment, the present invention relates to a conjugate of the antibody or fragment thereof of the present invention with a drug.

The drug can be any drug that has physiological activity. Examples of drugs include proteins, peptides, low-molecular compounds, nucleic acids, and sugar chains.

The method for conjugating the antibody or fragment thereof of the present invention with a drug is not particularly limited and can be any known method.

The conjugate of the present invention is formed by direct binding or indirect binding via a linker between the antibody of the present invention and a drug. The binding mode is not particularly limited and can be, for example, a covalent bond, coordination bond, or ionic bond. The antibody of the present invention and a drug can be bonded in accordance with or pursuant to a known method, depending on the binding mode.

The covalent bond is formed, for example, by allowing a functional group of the antibody of the present invention or its optionally introduced functional group to react with a functional group of a drug or its optionally introduced functional group. Examples of combinations of such functional groups include a combination between an amino group and a carboxyl group, a combination between a carboxyl group and a hydroxyl group, a combination between a maleimide group and a thiol group, a combination between a thiol group and a thiol group, a combination between a hydrazide group and a ketone group, a combination between a hydrazide group and an aldehyde group, a combination between an amino group and an aldehyde group, a combination between a thiol group and a carboxyl group, a combination between an amino group and a squaric acid derivative, a combination between a dienyl aldehyde group and an amino group, a combination between a halo ester and a thiol group, and a combination between azido and alkyne.

### 5. Use

In an embodiment, the present invention relates to a pharmaceutical composition, a reagent, or the like, containing at least one member selected from the group consisting of the antibody or fragment thereof of the present invention and the conjugate of the present invention.

The pharmaceutical composition of the present invention is suitable for use in the prevention or treatment of cancer, for use in the inhibition of vasculogenic mimicry in cancer, for use in the inhibition of periostin, for use in the prevention or treatment of inflammation-related diseases, for use in the inhibition of intimal hypertrophy, for use in the inhibition of angiogenesis, or for use in the prevention or treatment of aneurysm.

In an embodiment of the present invention, the antibody or fragment thereof of the present invention and the conjugate of the present invention can be used to prevent or treat inflammation-related diseases that involve periostin having cell adhesion activity.

The "inflammation-related diseases that involve periostin having cell adhesion activity" refer to diseases that exhibit high expression levels of the gene of periostin having cell adhesion activity and increased production of the protein encoded by the gene in pathological conditions. The inflammation-related diseases also refer to diseases the condition of which is aggravated by an increase in the gene or protein.

Such inflammation-related diseases with cell adhesion activity include, but are not particularly limited to, diseases of which the primary cause is intimal hypertrophy, cancers, and other inflammation-related diseases. Examples of diseases of which the primary cause is intimal hypertrophy include arteriosclerosis, and restenosis primarily caused by intimal hypertrophy observed after coronary angioplasty.

Examples of cancers to which the antibody or fragment thereof of the present invention and/or the conjugate of the present invention can be applied include, but are not limited to, brain tumor, leukemia, osteosarcoma, breast cancer, large intestine cancer, malignant melanoma, bone cancer, stomach cancer, lung cancer, liver cancer, kidney cancer, pancreas cancer, gallbladder cancer, skin cancer, uterus cancer, ovarian cancer, rectal cancer, colon cancer, fallopian tube cancer, esophageal cancer, small intestine cancer, thyroid cancer, parathyroid cancer, adrenal cancer, prostate cancer, bladder cancer, and malignant lymphoma; in particular, preferable examples include breast cancer, large intestine cancer, lung cancer, and malignant melanoma.

Other inflammation-related diseases include autoimmune arthritis, atopic dermatitis, asthma, emphysema, Behcet's disease, multiple sclerosis, spinocerebellar degeneration, uveitis, Guillain-Barre syndrome, Miller-Fisher syndrome, chronic inflammatory demyelinating polyneuropathy, polymyositis, scleroderma, autoimmune hepatitis, sarcoidosis, chronic pancreatitis, inflammatory enteritis, Crohn's disease, solid cancer, multiple myeloma, angiofibroma, atherosclerosis, arteriovenous malformation, granuloma, hemangioma, hypertrophic scar, keloid, progeria, psoriasis, pyrogenic granuloma, verruca, hemarthrosis, ununited fracture, rheumatoid arthritis (e.g., malignant rheumatoid arthritis), osteoarthritis, follicular cyst, ovarian hypertrophy syndrome, polycystic ovary syndrome, age-related macular degeneration, diabetic retinopathy, neovascular glaucoma, trachoma, emphysema, chronic bronchitis, obesity, periodontal disease, angiogenesis associated with corneal graft, and aneurysm. Angiogenesis is involved in many of these inflammation-related diseases.

Another embodiment of the present invention provides a diagnostic agent for inflammation-related diseases that involve periostin having cell adhesion activity, and the diagnostic agent is produced by labeling the antibody described above with a marker. The marker is not particularly limited, and examples thereof include enzymes, radioisotopes, and fluorescent dyes. The enzyme for use can be any enzyme that satisfies conditions such as having a high turnover number, remaining stable even after conjugation with an enzyme, and specifically reacting with their substrates to develop color. Enzymes for use in common enzyme immunoassay (EIA) are usable. Examples of preferred enzymes include peroxidases, β-galactosidases, alkaline phosphatases, glucose oxidase, acetylcholine esterase, glucose-6-phosphate dehydrogenase, and malate dehydrogenase. Enzyme inhibitors and coenzymes are also usable.

These enzymes can be conjugated with an antibody according to a known method using a known crosslinking agent, such as a maleimide compound. The substrate for use can be a known substance that is selected according to the enzyme for use. For example, for a peroxidase as an enzyme, 3,3',5,5'-tetramethylbenzidine can be used. For example, for an alkaline phosphatase as an enzyme, paranitrophenol can be used. The radioisotope for use as a marker can be those used in common radioimmunoassay (RIA), such as ¹²⁵I and ³H. The fluorescent dye for use can be those used in common fluorescence antibody techniques such as fluorescent isothiocyanate (FITC) and tetramethylrhodamine isothiocyanate (TRITC). The diagnostic agent can also be used in immunohistochemical staining that specifically stains cancer cells and surrounding fibroblasts. A diagnostic agent labeled with a radioisotope can be administered in vivo and used for imaging lesions of cancer etc.

Another embodiment of the present invention provides a method for detecting or quantifying periostin having cell adhesion activity in a biological sample (i.e., serum) prepared from human or animal blood, by using the antibody or fragment thereof of the present invention or the conjugate of the present invention. Another embodiment of the present invention further provides a method for diagnosing an inflammation-related disease in which periostin having cell adhesion activity is involved (e.g., heart failure), by detecting or quantifying periostin. The present methods can detect periostin having cell adhesion activity by using "sandwich ELISA" (enzyme-linked immunosorbent assay). In using a diagnostic kit of the present invention, a sample is first brought into contact with a plate on which an anti-periostin primary antibody is immobilized to conjugate the sample with the anti-periostin primary antibody, and then an anti-periostin secondary antibody labeled with a marker is allowed to bind to the conjugate. The signal intensity of the marker in the conjugate of these three components is measured to detect or quantify periostin having cell adhesion activity. Because periostin having cell adhesion activity is a splice variant that is specifically expressed in a pathological condition such as cancer, diagnosis of a pathological condition such as cancer can be made by monitoring the production of periostin.

As described above, the antibody of the present invention can be labeled and used as a secondary antibody.

Another embodiment of the present invention provides a pharmaceutical composition containing the antibody or fragment thereof of the present invention or the conjugate of the present invention as an active ingredient for inhibiting periostin having cell adhesion activity. Because the antibody, a fragment thereof, and/or a derivative thereof has inhibitory activity on periostin having cell adhesion activity, the pharmaceutical composition inhibits angiogenesis, inhibits intimal hypertrophy, treats cancer, or prevents or treats aneurysm. More specifically, the present invention provides a composition containing an anti-periostin antibody that recognizes a periostin splice variant having cell adhesion action, for inhibiting intimal hypertrophy, treating cancer, inhibiting angiogenesis, or preventing or treating aneurysm. The composition of the present invention can be used to treat and prevent restenosis that is primarily caused by intimal hypertrophy observed after coronary angioplasty, to prevent or treat cancer, to treat or prevent diseases accompanied by angiogenesis, and to prevent or treat aneurysm.

Another embodiment of the present invention provides a pharmaceutical composition containing the antibody of the present invention, a fragment thereof and/or a derivative thereof as an active ingredient for preventing or treating an inflammation-related disease that involves periostin having cell adhesion activity. An embodiment of the present invention provides, for example, a pharmaceutical composition containing the antibody, a fragment thereof, and/or a derivative thereof as an active ingredient for inhibiting intimal hypertrophy; a pharmaceutical composition containing the antibody, a fragment thereof, and/or a derivative thereof as an active ingredient for treating cancer; a pharmaceutical composition containing the antibody, a fragment thereof, and/or a derivative thereof as an active ingredient for inhibiting angiogenesis; and a pharmaceutical composition containing the antibody, a fragment thereof, and/or a derivative thereof as an active ingredient for preventing or treating aneurysm.

The composition of the present invention for treating cancer has the effects of inhibiting the growth of cancer foci and of inhibiting the metastasis of cancer. Thus, the composition can be used for a purpose of inhibiting the growth of cancer foci or of inhibiting the metastasis of cancer, or inhibiting both.

The pharmaceutical composition containing the antibody or fragment thereof of the present invention, or containing the conjugate of the present invention as an active ingredient is prepared by using known pharmacologically acceptable additives that are commonly used in a typical drug preparation method, including a carrier, an excipient, and other additives.

The active ingredient of the pharmaceutical composition according to the present invention is preferably mixed with known pharmacologically acceptable additives such as a carrier, an excipient, and a diluent and administered according to a method commonly used for pharmaceuticals (e.g., oral administration methods or parenteral administration methods such as intravenous, intramuscular, or subcutaneous administration). The pharmaceutical composition of the present invention can be produced, for example, by mixing the active ingredient with pharmacologically acceptable additives such as a carrier, a flavor, an excipient, a stabilizer, a diluent, an emulsifier, a solution, a suspension, and a syrup. Examples of dosage forms of the pharmaceutical composition of the present invention include, but are not particularly limited to, tablets, powders, granules, and solutions. Examples of additives that can be incorporated into tablets or the like include binders, such as gelatin, and lubricants, such as cornstarch. The pharmaceutical composition may be coated with sugar or a gastric soluble or enteric film. If the dosage form is a capsule, the composition may further contain a liquid carrier. The composition can also be prepared into an injectable sterile composition in accordance with a common pharmaceutical formula. Injectable aqueous vehicles include isotonic solutions containing glucose etc. Such isotonic solutions may be used in combination with an appropriate solubilizing agent, such as polyethylene glycol. The pharmaceutical composition may also be blended with a buffer, a stabilizer, a preservative, an antioxidant, a soothing agent, or the like. If the active ingredient is susceptible to degradation in the gastrointestinal tract in oral administration, the composition may be administered orally as a formulation that is less susceptible to degradation in the gastrointestinal tract (e.g., a liposome microcapsule containing the active ingredient). The pharmaceutical composition may also be administered by absorption through mucous membranes other than the gastrointestinal tract, such as in the rectum, nasal cavity, sublingual, and lungs. In this case, the composition may be administered in the form of a suppository, nasal spray, sublingual tablet, or transpulmonary formulation.

The dosage of the pharmaceutical composition of the present invention for use in treatment can be determined so as to be therapeutically effective. Because the therapeutically effective dosage varies, for example, according to the age, body weight and severity of the symptoms of a subject to which the composition is administered and the route of administration, the dosage is determined on an individual basis. In general, the daily dosage for an adult by oral administration is about 0.1 to 1000 mg, and the dosage may be given as a single dose or several divided doses (e.g., two or three times). In continuous intravenous administration, the composition can be administered at a dose of 0.01 µg/kg/min to 1.0 µg/kg/min, and preferably 0.025 µg/kg/min to 0.1 µg/kg/min.

Another embodiment of the present invention provides "a method for inhibiting periostin having cell adhesion activity by using an antibody against periostin" or "a method for preventing or treating an inflammation-related disease that involves periostin having cell adhesion activity by administering a therapeutically effective amount of an anti-periostin antibody that recognizes a periostin splice variant having cell adhesion action to a cancer patient." Another embodiment of the present invention provides "use of an antibody against periostin in the production of a pharmaceutical composition for inhibiting periostin having cell adhesion activity" or "use of an antibody against periostin in the production of a pharmaceutical composition for preventing or treating an inflammation-related disease that involves periostin having cell adhesion activity." The anti-periostin antibody includes the antibody, fragment thereof, and/or derivative thereof described above. The inflammation-related disease includes the inflammation-related diseases described above.

The reagent of the present invention may be in the form of a composition containing at least one member selected from the group consisting of the antibody or fragment thereof of the present invention and the conjugate of the present invention. The composition may optionally contain other components. Examples of other components include bases, carriers, solvents, dispersants, emulsifiers, buffers, stabilizers, excipients, binders, disintegrants, lubricants, thickeners, humectants, colorants, aroma components, and chelating agents.

The reagent of the present invention may be in the form of a kit containing at least one member selected from the group consisting of the antibody of the present invention and the conjugate of the present invention. The kit may contain instruments, reagents, etc. that are usable in detection or separation of human periostin. Examples of such instruments and reagents include test tubes, microtiter plates, agarose particles, latex particles, purification columns, labeling antibodies, and standard samples (positive and negative controls).

### Examples

The following describes the present invention in detail with reference to Examples. However, the present invention is not limited to these Examples.

### Test Example 1: Preparation of Monoclonal Antibody against Human Periostin

### (1) Preparation of Antigen

A peptide having a Cys residue added to the N-terminus of the amino acid sequence constituting human periostin exon 21 (SEQ ID: 65: EVTKVTKFIEGGDGHLFEDEEIKRLLQG) was chemically synthesized according to the Fmoc method to obtain 10 mg of an antigen peptide with a purity of 90% or higher. Then, 5 mg of a carrier protein KLH (CALBIOCHEM) was conjugated to 5 mg of the antigen peptide to obtain an antigen solution. Specifically, KLH was dissolved in PBS (0.01 M) to adjust the concentration to 3.3 mg/mL, and a 0.2524 mg/mL MBS solution (GE Healthcare BioSciences) was added dropwise thereto, followed by reacting the mixture with stirring at room temperature for 60 minutes. Free MBS was removed by using dichloromethane to obtain KLH-MB. Then, 5 mg of KLH-MB was mixed with a solution of 5 mg of the antigen peptide in 0.01 M sodium phosphate buffer (pH: 7.2), and the mixture was reacted with stirring at 4°C for 12 hours to obtain an antigen solution.

### (2) Immunization

The entirety of an emulsion mixture of 50 µl of the antigen solution containing 100 µg of the KLH-conjugated antigen peptide obtained in section (1) and 50 µl of FCA (Freund's complete adjuvant) was injected subcutaneously into both legs of three 6-week-old female BALB/c mice. Thereafter, an emulsion mixture of the antigen solution and FIA (Freund's incomplete adjuvant) was prepared on demand and administered to both legs twice at a 2-week interval. The mice were then killed by cervical dislocation, and the leg lymph nodes were aseptically harvested.

The lymph nodes were crushed with feeding of an RPMI medium (Kohjin Bio Co., Ltd.) and passed through a mesh with a pore diameter of about 10 um to obtain lymph node cells suspending in the RPMI medium. The suspension was subjected to centrifugation at 1000 rpm for 10 minutes to obtain a precipitation fraction of lymph node cells. 20 mM HEPES buffer (pH: 7.4) was added to a 0.84% ammonium chloride solution, and 1 ml of this solution was added to the precipitation fraction to hemolyze the fraction. After the red blood cells were removed, centrifugation was performed at 1,000 rpm for 5 minutes. The obtained precipitation fraction (cell fraction) was washed several times with an RPMI medium and used for cell fusion.

### (3) Preparation of Myeloma Cell

P3X63Ag8U.1, which is a 8-azaguanine-resistant and immunoglobulin-nonsecreting mouse myeloma cell line (P3U1 line), was cultured in an RPMI medium containing 20% fetal calf serum (FCS) in an incubator with 10% CO₂ at 37°C, and cells in the logarithmic growth phase were collected and centrifuged at 1,000 rpm for 5 minutes to obtain only cells as a precipitation fraction, followed by suspending the cells in an RPMI medium.

### (4) Fusion of Cell

The RPMI medium containing 1 × 10⁸ to 3 × 10⁸ immunized lymph node cells obtained in section (2) was mixed with the RPMI medium containing 10⁸ myeloma cells obtained in section (3) and then subjected to centrifugation at 1,000 rpm for 10 minutes. The supernatant was gently removed to obtain a precipitation fraction of cells, and 1 ml of 25% (w/v) polyethylene glycol 1500 (PEG 1500, Boehringer Ingelheim Co., Ltd.) was added thereto, followed by further adding an RPMI medium slowly to make the total volume 10 ml. To this, 10 ml of an RPMI medium containing 20% FCS was added, and the mixture was allowed to stand for a short time, followed by centrifugation at 1,000 rpm for 5 minutes. A cell suspension prepared by adding an RPMI medium containing 20% FCS to the obtained precipitation fraction (cell fraction) to adjust the cell concentration to 10⁶ cells/ml was dispensed into a 96-well culture plate (Corning Incorporated) in an amount of 200 µL/well. After incubation in an incubator with 5% CO₂ at 37°C for 24 hours, a HAT solution (Invitrogen Corporation) was added, and the cells were incubated for another 2 weeks.

### (5) Screening by ELISA

Screening was performed for wells positive for reaction between the culture supernatant and an antigen peptide. The antigen solution used for the assay was a conjugate prepared by binding 2 mg of the antigen peptide obtained in section (1), human periostin PN1 (full length), or mouse periostin PN2 (exon 17 deficient) to ovalbumin (OVA) as a carrier protein.

Each well of a 96-well microtiter plate (Falcon 353912) was coated with 1 ug/ml of the conjugate and left overnight at 4°C. After the plate was washed, 50 µl of the culture supernatant (containing a monoclonal antibody) of section (4) was added dropwise to each well, and the plate was left in an incubator at 37°C for 2 hours, followed by washing with PBS (-) (phosphate buffer). Alkaline phosphatase-conjugated sheep anti-mouse IgG antibody (Zymed) was added to the plate, which was then left in an incubator at 37°C for 1 hour and washed with PBS (-). Thereafter, a chromogenic substrate agent (ALP) was added to allow color to develop for 20 minutes, and the absorbance (antibody titer) at OD 490 nm in each well was measured with a plate reader (Bio-Rad, Model 680 Micro Plate Reader) to confirm reactivity with the antigen peptide and to determine the positive wells in which the culture supernatant reacted with the antigen peptide.

### (6) Cloning of Antibody-producing Cell

Antibody-producing cell lines were cloned according to a limiting dilution method from cells in wells confirmed to be positive for reactivity with the antigen peptide by ELISA in section (5). Specifically, cells in positive wells were seeded into each well of a 96-well culture plate and cultured for two weeks in 5% CO₂ at 37°C in an incubator. As with the method described in section (5), the reactivity of the culture supernatant of each well with the antigen peptide, human periostin PN1 (full length), or mouse periostin PN2 (exon 17 deletion) was confirmed according to ELISA. The cells in positive wells were cloned again according to the limiting dilution method to obtain cells that exhibited high reactivity with the antigen peptide and good development of cell colonies. These cells were transferred to a 24-well culture plate and cultured for 2 weeks in 5% CO₂ at 37°C in an incubator. From these cells, cells in 5 wells (i.e., 5 hybridoma cell lines A, B, C, D, and E) were selected. The selection was performed by using an immunoprecipitation test according to Test Example 2, described below.

### (7) Mass Preparation and Purification of Monoclonal Antibody

0.5 ml of pristane (2,6,10,14-tetramethylpentadecane, Fujifilm Wako Pure Chemical Corporation) was administered into the abdominal cavity of BALB/c mice, and the mice were reared for 2 to 3 weeks. Monoclonal antibody-producing hybridomas A to E that were maintained in the logarithmic growth phase beforehand were collected, and an FCS-free RPMI medium was added to the cells of the precipitation fraction, from which the culture supernatant was removed, to prepare a cell solution with a cell count of 1 × 10⁷/ml. The cell solution was injected into the abdominal cavity of the BALB/c mice to which pristane was pre-administered. Around 3 weeks later, leaked ascites was collected from the abdomen with a syringe. After filtration of the collected ascites with a filter with a pore diameter of 0.22 um, the filtrate was purified by affinity chromatography with a Protein G-Sepharose column (Millipore, 11511324) according to a common method, thereby preparing five anti-human-periostin-exon 21 monoclonal antibodies (antibodies A to E).

### Test Example 2: Human Periostin-Exon 21 Recognition Test 1

The monoclonal antibodies (antibodies A and B) obtained from hybridoma A and hybridoma B and antibody #8 reported to have been created in PTL 1 (antibody produced from an antibody-producing cell line (hybridoma) No. 8 (internationally deposited with the NITE Patent Microorganisms Depositary (NPMD), deposition date: February 26, 2013, accession number: NITE BP-01546, identification: KS-0259#8, 080611 Kohjin Bio) were used to perform a binding assay for human periostin secreted from human cells. The details of the assay are as follows.

After culturing Hs578t human breast cancer cells for 3 days in 0% FBS, immunoprecipitation was performed by using 1.0 ml of the supernatant. Antibody A, antibody B, antibody #8, and control (mouse IgG antibody) each in an amount of 5 µg were conjugated with immunoprecipitation beads (Invitrogen immunoprecipitation kit - Dynabeads Protein G Cat. #10007D), and incubated with the culture supernatant at 4°C overnight. Thereafter, the protein bound to each antibody was extracted, and western blotting was performed by using an antibody against periostin-exon 12 antigen (N-terminal antigen of periostin) as a primary antibody to confirm the binding of each exon 21 antibody to periostin.

Fig. 1 shows the results. Whereas antibody #8 did not recognize periostin-exon 21 secreted from the supernatant of human breast cancer cells at all, antibodies A and B strongly recognized periostin-exon 21 (Fig. 1(A)). After western blotting, CBB staining was performed to confirm that there was no bias in the antibody amount of each lane (Fig. 1(B)). The same test was performed on antibodies C to E, and antibodies C to E were also found to be able to recognize periostin-exon 21 secreted from the supernatant of human breast cancer cells.

### Test Example 3: Determination of CDR Sequence of Antibody

From the genome information of the hybridomas (A to E) cloned in Test Example 1, the amino acid sequences of antibodies A to E were determined. The amino acid sequences of the heavy-chain variable region and the light-chain variable region of antibodies A to E are as shown below (the sequence number is indicated in the parenthesis at the end of each sequence).

### Antibody A

### Heavy-chain Variable Region

KVQLVQSGAEVVKPGASVKLSCKASGYTFTEYIIHWVKQASGQGLEWIGWFYPGRGIIKYNEKFK
DKATLTADKSISTVYMDLSRLRSEDTAVYFCARHGITTATGAMDYWGQGTLVTVSS (SEQ ID
NO: 67) Light-chain Variable Region

DVVMTQTPLSLSVTLGQPASISCRSSQSLLHSNGNTYLHWYLQKPGQAPKLLIYKVSNRFSGVPD
RFSGSGSGTDFTLKISRVEAEDVGVYFCSQSTHVPFTFGQGTKLEIK (SEQ ID NO: 68).

### Antibody B

### Heavy-chain Variable Region

EVQLVESGGGLVKPGGSLRLSCAASGITFSSYAMSWVRQAPGKGLEWVASISSGGSTYYPDSVKG
RFTISRDNAKNSLYLQMNSLRAEDTAVYYCVAIWDGSSYGYWGQGTTVTVSS (SEQ ID NO:
69) Light-chain variable region

DIVMTQTPLSLSVTPGQPASISCKSGQSLVHSNGKTYLHWYLQKPGQSPQLLIYKVSNRFSGVPD
RFSGSGSGTDFTLKISRVEAEDVGVYYCSQSTHVPFTFGQGTKLEIK (SEQ ID NO: 70).

### Antibody C

### Heavy-chain Variable Region

EVQLVESGGGLVKPGGSLRLSCAASGFIFSSYAMSWVRQPPGKRLEWVASIGSGGTTYYPDSVRG
RFTISRDNARNILYLQMRSLRAEDTAVYYCAAIWDGSSHGYWGQGTLLTVSS (SEQ ID NO:
71)

### Light-chain variable region

DVVMTQSPLSLPVTLGQPASISCRSSQNLVHSNGNTYLHWYLQRPGQSPQLLIYKVSNRFSGVPD
RFSGSGSGTDFTLKISRVEAEDLGVYYCSQSTHVPYTFGPGTKLEIK (SEQ ID NO: 72).

### Antibody D

### Heavy-chain Variable Region

EVQLVESGGGLVKPGGSLRLSCAASGFTFSSYAMSWVRQTPEKRLEWVASINSGGVTTYYPDSVK
GRFTISRDNAINILYLQMRSLRSEDTALYYCAAIWDGSSHGYWGQGTLVTVSS (SEQ ID NO:
73)

### Light-chain variable region

DVVMTQSPLSLPVTLGQPASISCRSSQSLVHSNGNTYLHWYLQRPGQSPRLLIYKVSNRFSGVPD
RFSGSGSGTDFTLKISRVEAEDFGVYFCSQSTHVPYTFGPGTKLEIK (SEQ ID NO: 74).

### Antibody E

### Heavy-chain Variable Region

EVQLVESGGGLVQPGGSLKLSCAASGFTFSDAWMDWVRQAPGKGLEWVAEIRNKANNHATYFTES
VKGRFTISRDDSKNTVYLQMNSLRAEDTAVYYCTTATFVYWGQGTLVTVSS (SEQ ID NO:
75)

### Light-chain variable region

DVVMTQSPLSLPVSLGDRASISCRSSQSLVRNGITYLHWYQQKPGQSPKLLIYKVSNRFSGVPDR
FSGSGSGTDFTLKISSVEAEDFGVYYCSQTPHVPYTFGQGTKLEIK (SEQ ID NO: 76).

The amino acid sequences were compared with databases of the amino acid sequences of known antibodies (website: https://www.ncbi.nlm.nih.gov/igblast/ (IgBLAST) and website: http://www.imgt.org/ (IMGT)) to examine the homology, thereby determining the amino acid sequences of CDRs. Specifically, the "CDR sequences defined by the Kabat numbering scheme" were determined according to a comparison with the database in the website of IgBLAST, and the "CDR sequences defined by the IMGT numbering scheme" were determined according to a comparison with the database in the website of IMGT. Tables 1 to 5 show the CDR sequences defined by the Kabat numbering scheme (Kabat) and the CDR sequences defined by the IMGT numbering scheme (IMGT) of antibodies A to E.

**Table 1**

| | | Definition of CDR Sequence | Amino Acid Sequence | SEQ ID NO | | Definition of CDR Sequence | Amino Acid Sequence | SEQ ID NO |
|---|---|---|---|---|---|---|---|---|
| Antibody A | Heavy Chain CDR1 | Kabat | EYIIH | 1 | Light Chain CDR1 | Kabat | RSSQSLLHSNG NTYLH | 7 |
| | | IMGT | GYTFTEYI | 2 | | IMGT | QSLLHSNGNTY | 8 |
| | Heavy Chain CDR2 | Kabat | WFYPGRGIIKYN EKFKD | 3 | Light Chain CDR2 | Kabat | KVSNRFS | 9 |
| | | IMGT | FYPGRGII | 4 | | IMGT | KVS | 10 |
| | Heavy CDR3 | Kabat | HGITTATGAMDY | 5 | Light Chain CDR3 | Kabat | SQSTHVPFT | 11 |
| | | IMGT | ARHGITTATGAM DY | 6 | | IMGT | SQSTHVPFT | 12 |

**Table 2**

| | | Definition of CDR Sequence | Amino Acid Sequence | SEQ ID NO | | Definition of CDR Sequence | Amino Acid Sequence | SEQ ID NO |
|---|---|---|---|---|---|---|---|---|
| Antibody B | Heavy Chain CDR1 | Kabat | SYAMS | 13 | Light Chain CDR1 | Kabat | RSGQSLVHSNG KTYLH | 19 |
| | | IMGT | GITFSSYA | 14 | | IMGT | QSLVHSNGKTY | 20 |
| | Heavy Chain CDR2 | Kabat | SISSGGSTYYPD SVKG | 15 | Light Chain CDR2 | Kabat | KVSNRFS | 21 |
| | | IMGT | ISSGGST | 16 | | IMGT | KVS | 22 |
| | Heavy Chain CDR3 | Kabat | IWDGSSYGY | 17 | Light Chain CDR3 | Kabat | SQSTHVPFT | 23 |
| | | IMGT | VAIWDGSSYGY | 18 | | IMGT | SQSTHVPFT | 24 |

**Table 3**

| | | Definition of CDR Sequence | Amino Acid Sequence | SEQ ID NO | | Definition of CDR Sequence | Amino Acid Sequence | SEQ ID NO |
|---|---|---|---|---|---|---|---|---|
| Antibody C | Heavy Chain CDR1 | Kabat | SYAMS | 25 | Light Chain CDR1 | Kabat | RSSQNLVHSNG NTYLH | 31 |
| | | IMGT | GFIFSSYA | 26 | | IMGT | QNLVHSNGNTY | 32 |
| | Heavy Chain CDR2 | Kabat | SIGSGGTTYYPD SVRG | 27 | Light Chain CDR2 | Kabat | KVSNRFS | 33 |
| | | IMGT | IGSGGTT | 28 | | IMGT | KVS | 34 |
| | Heavy Chain CDR3 | Kabat | IWDGSSHGY | 29 | Light Chain CDR3 | Kabat | SQSTHVPYT | 35 |
| | | IMGT | AAIWDGSSHGY | 30 | | IMGT | SQSTHVPYT | 36 |

**Table 4**

| | | Definition of CDR Sequence | Amino Acid Sequence | SEQ ID NO | | Definition of CDR Sequence | Amino Acid Sequence | SEQ ID NO |
|---|---|---|---|---|---|---|---|---|
| Antibody D | Heavy Chain CDR1 | Kabat | SYAMS | 37 | Light Chain CDR1 | Kabat | RSSQSLVHSNG NTYLH | 43 |
| | | IMGT | GFTFSSYA | 38 | | IMGT | QSLVHSNGNTY | 44 |
| | Heavy Chain CDR2 | Kabat | SINSGGVTTYYP DSVKG | 39 | Light Chain CDR2 | Kabat | KVSNRFS | 45 |
| | | IMGT | INSGGVTT | 40 | | IMGT | KVS | 46 |
| | Heavy Chain CDR3 | Kabat | IWDGSSHGY | 41 | Light Chain CDR3 | Kabat | SQSTHVPYT | 47 |
| | | IMGT | AAIWDGSSHGY | 42 | | IMGT | SQSTHVPYT | 48 |

**Table 5**

| | | Definition of CDR Sequence | Amino Acid Sequence | SEQ ID NO | | Definition of CDR Sequence | Amino Acid Sequence | SEQ ID NO |
|---|---|---|---|---|---|---|---|---|
| Antibody E | Heavy Chain CDR1 | Kabat | DAWMD | 49 | Light Chain CDR1 | Kabat | RSSQSLVRNGIT YLH | 55 |
| | | IMGT | GFTFSDAW | 50 | | IMGT | QSLVRNGITY | 56 |
| | Heavy Chain CDR2 | Kabat | EIRNKANNHATY FTESVKG | 51 | Light Chain CDR2 | Kabat | KVSNRFS | 57 |
| | | IMGT | IRNKANNHAT | 52 | | IMGT | KVS | 58 |
| | Heavy Chain CDR3 | Kabat | ATFVY | 53 | Light Chain CDR3 | Kabat | SQTPHVPYT | 59 |
| | | IMGT | TTATFVY | 54 | | IMGT | SQTPHVPYT | 60 |

### Test Example 4: Determination of Epitope in Exon 21

For each of antibodies A to E, the epitope in human periostin-exon 21 was determined. The details are as follows.

Polypeptides, each having 10 amino acids from the N-terminus of the amino acid sequence of human periostin exon 21, were made. The polypeptides were arrayed on a glass slide. After blocking the array, primary antibodies A to E and Alexa Fluor 647-labeled secondary antibody were added sequentially to the peptide array and incubated. Antibodies bound to the epitope after a washing operation were detected by an Axon GenePix Scanner 4300 SL50. The fluorescence of each peptide spot was captured with a laser scanner, and positive peptides emitting a strong signal were analyzed with GenePix spot-recognition software.

The results indicated that antibodies A to E recognize the three-dimensional structure on human periostin-exon 21 containing the amino acid sequence A and the amino acid sequence B described in the following table.

**Table 6**

| | Amino Acid Sequence A (SEQ ID NO) | Amino Acid Sequence B (SEQ ID NO) |
|---|---|---|
| Antibody A | TKVTKF (61) | EIKRL (62) |
| Antibody B | TKVTKF (61) | DEEIKR (63) |
| Antibody C | TKVTKF (61) | DEEIKR (63) |
| Antibody D | TKVTKF (61) | DEEIKR (63) |
| Antibody E | TKVTKF (61) | IKRLLQ (64) |

### Test Example 5: Vasculogenic Mimicry Test 1

Research reports a phenomenon in which a tumor in cancer cells mimics blood vessels and allows blood flow in and out of the tumor. This phenomenon is called "vasculogenic mimicry" (Hendrix et al. Nature. 520, pp. 300-302 (2015)). This phenomenon has been observed in clinical cases such as breast cancer and reported as a poor prognostic factor. Research also reports the evaluation of lumen formation by cancer cells in a vasculogenic mimicry model of this cultured cell line. Thus, this culture model was used to compare antibodies.

Mouse breast cancer cells 4T07 were seeded onto a µ-Slide angiogenesis plate coated with Matrigel (Corning Matrigel Matrix Growth Factor Reduced 5 ml Cat. #356230) to induce vasculogenic mimicry. The culture medium for use was serum-free RPMI-1640, and the number of cells seeded was 3 × 10⁵ cells/ml. Mouse periostin (PN2) and periostin antibodies (antibody #8 and antibody A) were used as a drug to add. PN2 with a final concentration of 10 ug/ml and antibodies with a final concentration of 20 ug/ml were used, and these were incubated with 4T07 cells overnight. Thereafter, vasculogenic mimicry was analyzed. Analysis was performed with an Imaged Angiogenesis

### Analyzer.

Fig. 2 shows the results. The administration of PN2 markedly induced vasculogenic mimicry in breast cancer cells (P<0.05). Whereas the administration of antibody #8 in addition to PN2 did not decrease the indicators of lumen formation, such as the number of nodes and the number of segments, the use of antibody A in addition to PN2 significantly decreased these indicators (P<0.01).

### Test Example 6: Cancer Cell Transplantation Test

Mouse breast cancer cells 4T07 were seeded in tissue culture treated 10 cm dishes (Greiner) by using RPMI 1640 (Gibco) supplemented with 10% bovine serum albumin (Invitrogen) and Penicillin-Streptomycin Mixed Solution (Nacalai Tesque), and incubated at 37°C in an incubator for 24 hours. Thereafter, the culture supernatant was removed, and the dishes were washed with PBS, after which the cells were allowed to float with trypsin /EDTA. The cells were then collected and centrifuged for 5 minutes, and counted to make 1 × 10⁶ breast cancer cells per animal, followed by suspending the cells in 100 µl of PBS. The conditioned cells were transplanted into the mammary gland of 8-week-old female BALB/c nude mice by using a 25G injection needle. From one week after transplantation, the diameter (mm) and short diameter (mm) of the mammary gland site where the cells had grown were measured with a caliper, and the tumor volume was calculated according to the following formula: tumor volume = diameter × short diameter² × 0.5. When the volume reached 100 mm³, the mice were divided into three groups: a control group, a group administered with antibody #8, and a group administered with antibody A (6 mice for each group). Subsequently, the antibodies were administered via tail vein by using a 30G injection needle at 10 mg/kg, twice per week. Thereafter, the tumor volume was compared.

Fig. 3 shows the results. Whereas the group administered with antibody #8 showed no tumor shrinkage compared with the control group, the group administered with antibody A showed a marked tumor growth suppression effect (P<0.01).

### Test Example 7: Human Periostin-Exon 21 Recognition Test 2

A binding assay for human periostin secreted from human cells was performed with antibodies A to E in the same manner as in Test Example 2.

Fig. 4 shows the results. As shown in Fig. 4, the results indicate that not only antibodies A and B but also antibodies C to E can recognize periostin-exon 21 secreted from cells. This makes it even more understandable that the antibody of the present invention can recognize periostin-exon 21 secreted from cells.

### Test Example 8: Vasculogenic Mimicry Test 2

Antibodies A, B, C, and D were evaluated in terms of the lumen formation by cancer cells in vasculogenic mimicry models in the same manner as in Test Example 5. However, the total master segments length was measured as an indicator of lumen formation.

Fig. 5 shows the results. As shown in Fig. 5, the results indicate that not only antibody A but also antibodies B, C, and D can suppress the lumen formation by cancer cells in vasculogenic mimicry models. This makes it even more understandable that the antibody of the present invention has action of inhibiting vasculogenic mimicry.

### Test Example 9: Vasculogenic Mimicry Test 3

Antibodies A, B, C, and D were evaluated in terms of the lumen formation by cancer cells in vasculogenic mimicry models in the same manner as in Test Example 5. However, the total mesh area (the total area of formed lumina) was measured as an indicator of lumen formation.

Fig. 6 shows the results. As shown in Fig. 6, the results indicate that not only antibody A but also antibody E can suppress the lumen formation by cancer cells in vasculogenic mimicry models.

## Claims

1. An antibody or antigen-binding fragment thereof bindable to a conformational epitope on human periostin,
the epitope comprising the amino acid sequence A represented by SEQ ID NO: 61 and the amino acid sequence B represented by any one of SEQ ID NOs: 62 to 64.

2. The antibody or antigen-binding fragment thereof according to claim 1, wherein the human periostin is human periostin secreted from a human cell.

3. The antibody or antigen-binding fragment thereof according to claim 1 or 2, wherein the human cell is a human breast cancer cell.

4. The antibody or antigen-binding fragment thereof according to any one of claims 1 to 3, which is a monoclonal antibody or antigen-binding fragment thereof.

5. The antibody or antigen-binding fragment thereof according to any one of claims 1 to 4, comprising
heavy-chain CDR 1 comprising the amino acid sequence represented by SEQ ID NO: 1 and/or 2, SEQ ID NO: 13 and/or 14, SEQ ID NO: 25 and/or 26, SEQ ID NO: 37 and/or 38, or SEQ ID NO: 49 and/or 50,
heavy-chain CDR 2 comprising the amino acid sequence represented by SEQ ID NO: 3 and/or 4, SEQ ID NO: 15 and/or 16, SEQ ID NO: 27 and/or 28, SEQ ID NO: 39 and/or 40, or SEQ ID NO: 51 and/or 52,
heavy-chain CDR 3 comprising the amino acid sequence represented by SEQ ID NO: 5 and/or 6, SEQ ID NO: 17 and/or 18, SEQ ID NO: 29 and/or 30, SEQ ID NO: 41 and/or 42, or SEQ ID NO: 53 and/or 54,
light-chain CDR 1 comprising the amino acid sequence represented by SEQ ID NO: 7 and/or 8, SEQ ID NO: 19 and/or 20, SEQ ID NO: 31 and/or 32, SEQ ID NO: 43 and/or 44, or SEQ ID NO: 55 and/or 56,
light-chain CDR 2 comprising the amino acid sequence represented by SEQ ID NO: 9 and/or 10, SEQ ID NO: 21 and/or 22, SEQ ID NO: 33 and/or 34, SEQ ID NO: 45 and/or 46, or SEQ ID NO: 57 and/or 58, and
light-chain CDR 3 comprising the amino acid sequence represented by SEQ ID NO: 11 and/or 12, SEQ ID NO: 23 and/or 24, SEQ ID NO: 35 and/or 36, SEQ ID NO: 47 and/or 48, or SEQ ID NO: 59 and/or 60.

6. The antibody or antigen-binding fragment thereof according to any one of claims 1 to 5, comprising the following (A), (B), (C), (D), or (E) :
(A)
heavy-chain CDR 1 comprising the amino acid sequence represented by SEQ ID NO: 1 and/or 2,
heavy-chain CDR 2 comprising the amino acid sequence represented by SEQ ID NO: 3 and/or 4,
heavy-chain CDR 3 comprising the amino acid sequence represented by SEQ ID NO: 5 and/or 6,
light-chain CDR 1 comprising the amino acid sequence represented by SEQ ID NO: 7 and/or 8,
light-chain CDR 2 comprising the amino acid sequence represented by SEQ ID NO: 9 and/or 10, and
light-chain CDR 3 comprising the amino acid sequence represented by SEQ ID NO: 11 and/or 12;
(B)
heavy-chain CDR 1 comprising the amino acid sequence represented by SEQ ID NO: 13 and/or 14,
heavy-chain CDR 2 comprising the amino acid sequence represented by SEQ ID NO: 15 and/or 16,
heavy-chain CDR 3 comprising the amino acid sequence represented by SEQ ID NO: 17 and/or 18,
light-chain CDR 1 comprising the amino acid sequence represented by SEQ ID NO: 19 and/or 20,
light-chain CDR 2 comprising the amino acid sequence represented by SEQ ID NO: 21 and/or 22, and
light-chain CDR 3 comprising the amino acid sequence represented by SEQ ID NO: 23 and/or 24;
(C)
heavy-chain CDR 1 comprising the amino acid sequence represented by SEQ ID NO: 25 and/or 26,
heavy-chain CDR 2 comprising the amino acid sequence represented by SEQ ID NO: 27 and/or 28,
heavy-chain CDR 3 comprising the amino acid sequence represented by SEQ ID NO: 29 and/or 30,
light-chain CDR 1 comprising the amino acid sequence represented by SEQ ID NO: 31 and/or 32,
light-chain CDR 2 comprising the amino acid sequence represented by SEQ ID NO: 33 and/or 34, and
light-chain CDR 3 comprising the amino acid sequence represented by SEQ ID NO: 35 and/or 36;
(D)
heavy-chain CDR 1 comprising the amino acid sequence represented by SEQ ID NO: 37 and/or 38,
heavy-chain CDR 2 comprising the amino acid sequence represented by SEQ ID NO: 39 and/or 40,
heavy-chain CDR 3 comprising the amino acid sequence represented by SEQ ID NO: 41 and/or 42,
light-chain CDR 1 comprising the amino acid sequence represented by SEQ ID NO: 43 and/or 44,
light-chain CDR 2 comprising the amino acid sequence represented by SEQ ID NO: 45 and/or 46, and
light-chain CDR 3 comprising the amino acid sequence represented by SEQ ID NO: 47 and/or 48; or
(E)
heavy-chain CDR 1 comprising the amino acid sequence represented by SEQ ID NO: 49 and/or 50,
heavy-chain CDR 2 comprising the amino acid sequence represented by SEQ ID NO: 51 and/or 52,
heavy-chain CDR 3 comprising the amino acid sequence represented by SEQ ID NO: 53 and/or 54,
light-chain CDR 1 comprising the amino acid sequence represented by SEQ ID NO: 55 and/or 56,
light-chain CDR 2 comprising the amino acid sequence represented by SEQ ID NO: 57 and/or 58, and
light-chain CDR 3 comprising the amino acid sequence represented by SEQ ID NO: 59 and/or 60.

7. The antibody or antigen-binding fragment thereof according to any one of claims 1 to 6, comprising (A)
heavy-chain CDR 1 comprising the amino acid sequence represented by SEQ ID NO: 1 and/or 2,
heavy-chain CDR 2 comprising the amino acid sequence represented by SEQ ID NO: 3 and/or 4,
heavy-chain CDR 3 comprising the amino acid sequence represented by SEQ ID NO: 5 and/or 6,
light-chain CDR 1 comprising the amino acid sequence represented by SEQ ID NO: 7 and/or 8,
light-chain CDR 2 comprising the amino acid sequence represented by SEQ ID NO: 9 and/or 10, and
light-chain CDR 3 comprising the amino acid sequence represented by SEQ ID NO: 11 and/or 12.

8. An antibody obtained by a method comprising selecting an antibody bindable to human periostin secreted from a human breast cancer cell from among antibodies produced by a hybridoma obtained from an animal immunized with at least one protein or peptide selected from the group consisting of:
(e) a protein or peptide containing the amino acid sequence represented by SEQ ID NO: 65, and
(f) a protein or peptide containing an amino acid sequence X with at least 85% identity to the amino acid sequence represented by SEQ ID NO: 65 and containing the amino acid sequence A represented by SEQ ID NO: 61 and the amino acid sequence B represented by any one of SEQ ID NOs: 62 to 64 within the amino acid sequence X.

9. An antibody produced from a hybridoma obtained from an animal immunized with at least one protein or peptide selected from the group consisting of:
(e) a protein or peptide containing the amino acid sequence represented by SEQ ID NO: 65, and
(f) a protein or peptide containing an amino acid sequence X with at least 85% identity to the amino acid sequence represented by SEQ ID NO: 65 and containing the amino acid sequence A represented by SEQ ID NO: 61 and the amino acid sequence B represented by any one of SEQ ID NOs: 62 to 64 within the amino acid sequence X,
the antibody being bindable to human periostin secreted from a human breast cancer cell.

10. A polynucleotide comprising a coding sequence of the antibody or antigen-binding fragment thereof of any one of claims 1 to 9.

11. A cell comprising the polynucleotide of claim 10.

12. A conjugate of the antibody or antigen-binding fragment thereof of any one of claims 1 to 9 with a drug.

13. A pharmaceutical composition comprising at least one member selected from the group consisting of the antibody or antigen-binding fragment thereof of any one of claims 1 to 9 and the conjugate of claim 12.

14. The pharmaceutical composition according to claim 13, for use in the prevention or treatment of cancer, for use in the inhibition of vasculogenic mimicry in cancer, for use in the inhibition of periostin, for use in the prevention or treatment of inflammation-related diseases, for use in the inhibition of intimal hypertrophy, for use in the inhibition of angiogenesis, or for use in the prevention or treatment of aneurysm.

15. A reagent comprising at least one member selected from the group consisting of the antibody or antigen-binding fragment thereof of any one of claims 1 to 9 and the conjugate of claim 12.
